(19) Europäisches Patentamt
European Patent Office
Office européen des brevets

(11) EP 3 134 103 B1

(12) EUROPEAN PATENT SPECIFICATION

(45) Date of publication and mention of the grant of the patent:
**15.01.2020 Bulletin 2020/03**

(21) Application number: **15783438.3**

(22) Date of filing: **27.04.2015**

(51) Int Cl.:
***A61K 38/07*** $^{(2006.01)}$ ***A61K 9/08*** $^{(2006.01)}$
***A61P 25/00*** $^{(2006.01)}$ ***A61K 47/02*** $^{(2006.01)}$
***A61K 47/18*** $^{(2017.01)}$

(86) International application number:
**PCT/US2015/027745**

(87) International publication number:
**WO 2015/164859 (29.10.2015 Gazette 2015/43)**

# (54) STABLE COMPOSITIONS OF NEUROACTIVE PEPTIDES

STABILE ZUSAMMENSETZUNGEN VON NEUROAKTIVEN PEPTIDEN

COMPOSITIONS STABLES DE PEPTIDES NEUROACTIFS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **25.04.2014 US 201461984216 P**

(43) Date of publication of application:
**01.03.2017 Bulletin 2017/09**

(73) Proprietor: **Naurex Inc.**
**Evanston, Illinois 60201 (US)**

(72) Inventors:
- **HOUCK, David, Renwick**
**Cary, NC 27159 (US)**
- **ARGHAVANI, Mohsen**
**Lake in the Hills, IL 60156 (US)**

(74) Representative: **Hoffmann Eitle**
**Patent- und Rechtsanwälte PartmbB**
**Arabellastraße 30**
**81925 München (DE)**

(56) References cited:
**WO-A1-2011/100585 WO-A2-2013/188465**
**US-A1- 2011 098 213 US-A1- 2012 178 695**

- **JOSEPH R MOSKAL ET AL: "GLYX-13, an NMDA receptor glycine site functional partial agonist enhances cognition and produces antidepressant effects without the psychotomimetic side effects of NMDA receptor antagonists", EXPERT OPINION ON INVESTIGATIONAL DRUGS, vol. 23, no. 2, 20 November 2013 (2013-11-20), pages 243-254, XP055380018, UK ISSN: 1354-3784, DOI: 10.1517/13543784.2014.852536**
- **JEFFREY BURGDORF ET AL: "ACCEPTED ARTICLE PREVIEW - GLYX-13, an NMDA Receptor Glycine-Site Functional Partial Agonist, Induces Antidepressant-Like Effects Without Ketamine-Like Side Effects", INTERNET CITATION, 3 December 2012 (2012-12-03), pages 1-46, XP002691138, Retrieved from the Internet: URL:http://www.nature.com/npp/journal/vaop /naam/pdf/npp2012246a.pdf [retrieved on 2013-01-30]**

EP 3 134 103 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

BACKGROUND

The central nervous system (CNS) of mammals employs many neuroactive peptides to effect specialized signaling within the brain and spinal cord including the neuroactive peptides somatostatin, cholecystokinin, VIP, Substance P, enkephalin, Neuropeptide Y (NPY), Neurotensin, TRH, CCK, and dynorphin. (The Biochemical Basis of Neuropharmacology, Cooper, Bloom and Roth, 5th ed., Oxford University Press, New York, 1986). The careful elucidation of the complex signaling pathways, which operate in the CNS, has led to identification of specific receptors modulated by these neuroactive peptides presenting important therapeutic targets for various disorders associated with the CNS.

An N-methyl-D-aspartate (NMDA) receptor (NMDAR) is one such receptor that has drawn particular interest since it appears to be involved in a broad spectrum of CNS disorders. The NMDAR has been implicated in neurodegenerative disorders including stroke-related brain cell death, convulsive disorders, and learning and memory. NMDAR also plays a central role in modulating normal synaptic transmission, synaptic plasticity, and excitotoxicity in the central nervous system. The NMDAR is further involved in Long-term potentiation (LTP). LTP is the persistent strengthening of neuronal connections that underlie learning and memory (See Bliss and Collingridge, 1993, Nature 361:31-39).

Several compounds have been identified which exert a dual (agonist/antagonist) effect on the NMDA receptor through the allosteric sites. These compounds are termed "partial agonists". In the presence of the principal site ligand, a partial agonist will displace some of the ligand and thus decrease $Ca^{++}$ flow through the receptor. In the absence of or lowered level of the principal site ligand, the partial agonist acts to increase $Ca^{++}$ flow through the receptor channel.

Recently, an improved partial agonist of NMDAR, termed as GLYX-13 or rapastinel, has been reported. GLYX-13 is exemplified by the following structure:

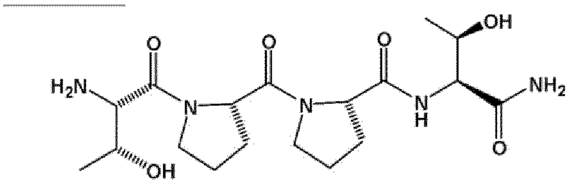

,

with a molecular weight: 413.47, and a chemical formula: $C_{18}H_{31}N_5O_6$. GLYX-13 exhibits nootropic, neuroprotective and antinociceptive activity, and enhances learning, memory and cognition in vivo.

Although a number of therapeutic benefits of GLYX-13 have already been elucidated, there still remains a need for ways to efficiently deliver GLYX-13 so as to ensure GLYX-13 effectively crosses the blood brain barrier and is efficiently absorbed at the required site of action to effect improved treatment of CNS disorders like depression, neuropathic pain, or anxiety. Additionally, it would be desirable that the delivery formulation is stable (i.e., not subject to degradation) in an aqueous media.

SUMMARY

The invention is as defined in the present claims. This disclosure features stable aqueous compositions suitable for intravenous injection that include a GLYX peptide having the following formula:

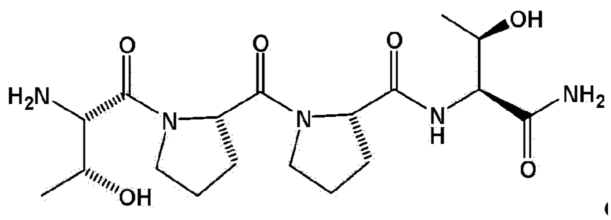

,

or a salt thereof. Embodiments can include one or more of the following advantages. In some embodiments, the compositions described herein can enhance clinical delivery or administration of the GLYX-13 peptide and/or salt thereof to the circulatory system by IV injection, thereby resulting in more efficient delivery of the GLYX peptide to the brain, and thus to one or more active sites in the brain associated with treatment of CNS disorders such as depression, neuropathic pain, or anxiety. In some embodiments, the compositions described herein can exhibit enhanced storage stability, e.g., rendering the GLYX peptides less susceptible to degradation in aqueous media. The compositions described herein

can further include one or more pharmaceutically acceptable moieties (e.g., including a buffer, a water miscible solvent, an excipient, pharmaceutically acceptable anions (e.g., chloride ion) and cations (e.g., $H^+$)) that confer one or more physical and/or chemical properties to the compositions. For example, the one or more pharmaceutically acceptable moieties can be an acid (e.g., hydrochloric acid, e.g., dissociated hydrochloric acid), its conjugate base (sometimes referred to herein as a "buffer," e.g., chloride ion), or a combination thereof and can be present in amounts sufficient to maintain a particular pH. It will also be appreciated by those skilled in the art that the compositions described herein may additionally contain, if desired, a combination of two or more active additional ingredients.

(1) In one embodiment, the present invention features a stable, aqueous composition suitable for intravenous injection, comprising (i) 60 mg/mL to 200 mg/mL of a pharmaceutically active compound having the formula:

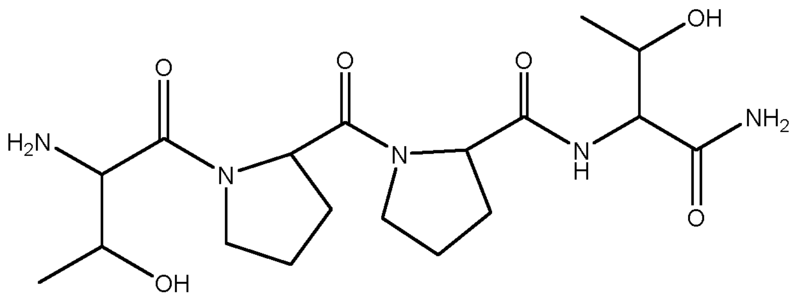

;

or a pharmaceutically acceptable salt thereof; (ii) water for injection; and (iii) an acid; wherein the stable, aqueous composition has a pH of from 3.9 to 5.5 at 25 °C; and wherein the acid provides chloride ions in the aqueous composition.

(2) The stable, aqueous composition of embodiment 1, comprising from 75 mg/mL, 150 mg/mL, or from 125 mg/mL to 175 mg/mL of the pharmaceutically active compound.

(3) The stable, aqueous composition of any one of embodiments 1-2, comprising 200 mg to 500 mg of the pharmaceutically active compound, preferably 225 mg, 375 mg or 450 mg of the pharmaceutically active compound.

(4) The stable, aqueous composition of any one of embodiments 1-3, wherein the stable, aqueous composition has a pH of about 4.5 at 25 °C.

(5) The stable, aqueous composition of any one of embodiments 1-4 comprising at least one of: H+, a protonated form of the pharmaceutically active compound, and/or a combination thereof.

(6) The stable, aqueous composition of any one of embodiments 1-5, wherein upon administration of a dose of the stable, aqueous liquid composition that comprises 150 mg/mL of the pharmaceutically active compound and has a volume of 3 mL to a patient, a physiological osmolality of from 800 mOsmol/kg to 900 mOsmol/kg is obtained in said patient.

(7) The stable, aqueous composition of any one of embodiments 1-5, upon administration of a dose of the stable, aqueous liquid composition that comprises 75 mg/mL of the pharmaceutically active compound and has a volume of 3 mL to a patient, a physiological osmolality of from 375 mOsmol/kg to 475 mOsmol/kg is obtained in said patient.

(8) The stable, aqueous composition of any one of embodiments 1-7, wherein the composition has a minimal amount of one or more of degradation products each selected from the group consisting of cyclo proline-threonine (diketopiperazine), Thr-Pro-Pro-Thr, Pro-Pro-Thr, Pro-Pro-Thr-NH2, Thr-Pro, Pro-Thr, Pro-Thr-NH2, proline and/or threonine after 10 days at room temperature, after 20 days at room temperature or after 1 month at 0 °C or below.

(9) The stable, aqueous composition of any one of embodiments 1-8, wherein the composition has less than about 2% area obtained by HPLC of the GLYX -13 peak of diketopiperazine and/or Pro-Thr-NH2 after 3 months at 40 °C, preferably less than about 1% or less than about 0.5% area obtained by HPLC of the GLYX -13 peak by HPLC of diketopiperazine and/or Pro-Thr-NH2 after 3 weeks at 40 °C.

(10) A receptacle containing an amount of the stable, aqueous composition of any one of embodiments 1-9, extractable as at least one single dose.

(11) The receptacle of claim 10, wherein the single dose has a volume of 1 mL to 4 mL, and preferably has a volume of 3 mL.

(12) A pre-filled syringe or vial comprising a single dose of the stable, aqueous liquid composition of any one of embodiments 1-11.

(13) A stable, aqueous composition according to anyone of embodiments 1-9 comprising:
about 150 mg/mL of a compound represented by:

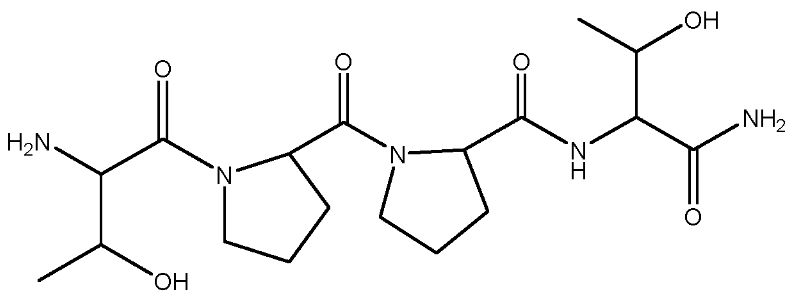

water for injection; and hydrochloric acid, wherein the composition has a pH of 4.1 to 4.7 at 25°C.

(14) A pharmaceutically acceptable dose suitable for injection comprising: 225 mg or 450 mg of a compound represented by:

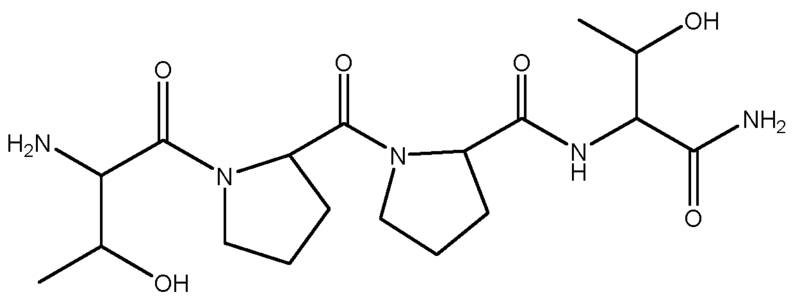

water; and
an acid providing chloride ions in the aqueous composition, wherein the dose has a pH of 4.5 and a volume of 3 mL.

(15) The dose of embodiment 14, wherein the dose is disposed within a syringe or a vial.

(16) The composition according to any one of embodiments 1-9 or 13, wherein the composition is prepared by a process comprising:

(i) providing a first combination comprising the pharmaceutically active compound and water; and
(ii) contacting the first combination with hydrochloric acid, or a source thereof, in an amount sufficient to achieve a pH of from 3.9 to 5.5.

(17) The composition according to any one of embodiments 1-9 or 13 for the use of treating

In another aspect, this disclosure features a receptacle (e.g., a prefilled syringe or vial) containing an amount of any of the stable, aqueous compositions described herein. In certain embodiments, the amount is extractable as at least one single dose. In certain embodiments, the single dose can have a volume of 1 mL to 4 mL (e.g., 3 mL).
In a further aspect, this disclosure features a pre-filled syringe that includes a single dose of any of the stable, aqueous compositions described herein. In certain embodiments, the single dose can have a volume of 1 mL to 4 mL (e.g., 3 mL).

BRIEF DESCRIPTION OF THE FIGURES

Figure 1 shows the formation of impurity (% area proline-threonine diketopiperazine, RRT 0.43) over time versus pH of a disclosed composition

Figure 2 shows the formation of impurity (% area proline-threonine-amide, RRT 0.57) over time versus pH of a disclosed composition.

DETAILED DESCRIPTION

This disclosure is directed in part to a stable, aqueous compositionthat is suitable for intravenous injection 60 mg/mL to 200 mg/mL of a pharmaceutically active compound having the following formula:

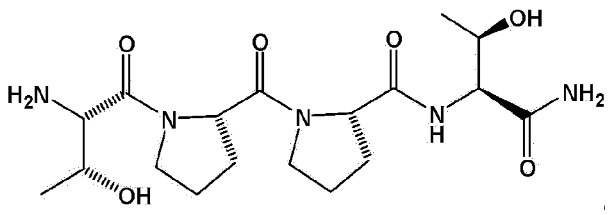

or a pharmaceutically acceptable salt thereof water for injection; and an acid; wherein the stable, aqueous composition has a pH of from 3.9 to 5.5 at 25 °C; and wherein the acid provides chloride ions in the aqueous composition.

The disclosed compositions can include one or more of the following advantages. In some embodiments, the compositions described herein can enhance clinical delivery or administration of the GLYX-13 peptide and/or salt thereof; to the circulatory system by IV injection, thereby resulting in more efficient delivery of the GLYX peptide as defined above to the brain, and thus to one or more active sites in the brain associated with treatment of CNS disorders such as depression, neuropathic pain, or anxiety. In some embodiments, the compositions described herein can exhibit enhanced storage stability, e.g., rendering the GLYX peptides as defined above less susceptible to degradation in aqueous media. The compositions described herein caninclude one or more pharmaceutically acceptable substances (e.g., including but not limited to, a buffer, a water miscible solvent and/or an excipient) that confer one or more physical and/or chemical properties to the disclosed compositions. For example, the one or more pharmaceutically acceptable substances that may form part of a contemplated composition can be selected from the group consisting of an acid (e.g., hydrochloric acid), its conjugate base (sometimes referred to herein as a "buffer," e.g., chloride ion), or a combination thereof and can be present in amounts sufficient to maintain a particular pH. It will also be appreciated by those skilled in the art that the compositions described herein may additionally contain, if desired, a combination of two or more active additional ingredients.

The chemical and physical stability and/or the pharmaceutical acceptability of disclosed compositions may be determined by techniques well known to those skilled in the art. Thus, for example, the chemical stability of the components may be determined by HPLC assay and/or color by appearance, for example, after prolonged storage of the product. Physical stability data may be gained from other conventional analytical techniques.

**Peptides**

As used herein, the term "GLYX peptide" refers to a peptide having NMDAR glycine-site partial agonist/antagonist activity. GLYX peptides may be obtained by well-known recombinant or synthetic methods such as those described in US Patents 5,763,393 and 4,086,196. Exemplary GLYX peptides contemplated as forming part of disclosed formulations mayinclude one or more of the listed peptides of Table 1:

TABLE 1

| Name SEQ ID NO | Amino Acid Sequence |
|---|---|
| NT-1: SEQ ID. NO:1. | Lys-Ala-Ser-Gln-Asp-Val-Ser-Thr-Thr-Val-Ala |
| NT-2: SEQ ID. NO:2. | Ser-Ala-Ser-Tyr-Arg-Tyr-Thr |
| NT-3: SEQ ID. NO:3. | Gln-Gln-His-Tyr-Ser-Thr-Pro-Pro-Thr |

(continued)

| Name SEQ ID NO | Amino Acid Sequence |
|---|---|
| NT-4: SEQ ID. NO:4. | Val—Tyr—Tyr—Ser—Gln—Gln—His—Tyr—Ser—Thr—Pro—Pro—Thr |
| NT-5: SEQ ID. NO:5. | Glu—Asp—Leu—Ala—Val—Tyr—Tyr—Ser—Gln—Gln—His—Tyr—Ser—Thr—Pro—Pro—Thr |
| NT-6: SEQ ID. NO:6. | Ser—Val—Gln—Ala—Glu—Leu—Asp—Leu—Ala—Val—Tyr—Tyr—Ser—Gln—Gln—His—Tyr—Ser—Thr—Pro—Pro—Thr |
| NT-7: SEQ ID. NO:7. | Phe—Thr—Ile—Ser—Ser—Val—Gln—Ala—Glu—Leu—Asp—Leu—Ala—Val—Tyr—Tyr—Ser—Gln—Gln—His—Tyr—Ser—Thr—Pro—Pro—Thr |
| NT-8: SEQ ID. NO: 8. | Gln—Gln—His—Tyr—Ser—Thr—Pro—Pro—Thr—Phe—Gly—Gly—Gly |
| NT-9: SEQ ID. NO:9. | Gln—Gln—His—Tyr—Ser—Thr—Pro—Pro—Thr—Phe—Gly—Gly—Gly—Thr—Lys—Leu—Glu |
| NT-10: SEQ ID. NO: 10 | Cys—Gln—Gln—His—Tyr—Ser—Thr—Pro—Pro—Thr—Cys<br>S—S |
| NT-11: SEQ ID. NO:11 | Ser-Gln-Gln-His-Tyr-Ser-Thr-Pro-Pro-Thr-Ser |
| NT-12: SEQ ID. NO:12 | Gln-Gln-His-Tyr-Ser |
| NT-13: SEQ ID. NO:13 | Thr-Pro-Pro-Thr |
| NT-14: SEQ ID. NO:14 | Thr-Pro-Pro |
| NT-15: SEQ ID. NO:15 | Pro-Pro-Thr |
| NT-16: SEQ ID. NO:16 | Pro-Pro |
| NT-17: SEQ ID. NO:17 | Thr-Pro-Thr |
| NT-18: SEQ ID. NO:18 | Thr |

"GLYX-13" is represented by the following formula:

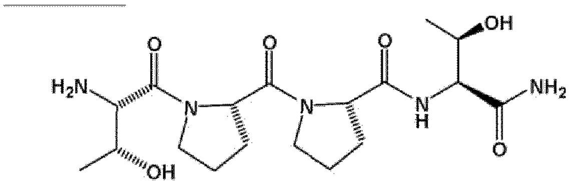

and/or pharmaceutically acceptable salt forms of the above compound.

For example, disclosed compositions may prove effective for use in the treatment of many neurological diseases, such as Alzheimer's, Parkinson's, psychiatric diseases and intracerebral infections.

In some embodiments, the compositions described herein have a pH of from 3.9 to 5.5, from at 25 °C. The compositions described herein may have a pH of from 4.2 to 5.0 (e.g., from 4.2 to 4.8, from 4.3 to 4.7, from 4.4 to 4.6). For example, disclosed compositions in certain embodiments as described herein have a pH of 4.5 at 25 °C.

In some embodiments, the compositions described herein can include less than 100 mg/mL of a GLYX-13 peptide having the following formula:

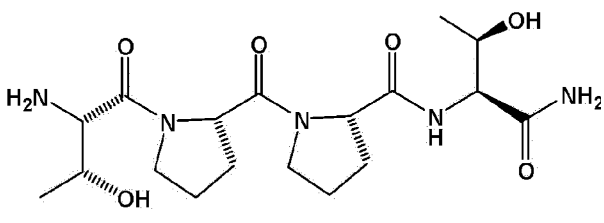

or a pharmaceutically acceptable salt thereof). The compositions described herein include 60 mg/mL of the GLYX-13

peptide and/or pharmaceutically acceptable salt thereof.

In some embodiments, the compositions described herein comprise a GLYX peptide having the following formula:

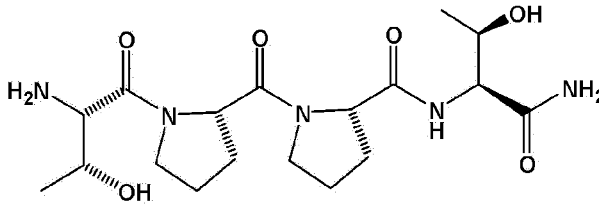

or a pharmaceutically acceptable salt thereof from 75 mg/mL, 150 mg/mL, or from 125 mg/mL to 175 mg/ of the pharmaceutically active compound In certain embodiments, the compositions described herein have a pH of 4.5 at 25 °C

In some embodiments, the compositions described herein can have from 200 mg to 500 mg,preferably 225 mg, 375 mg or 450 mg of the GLYX-13 peptide having the following formula:

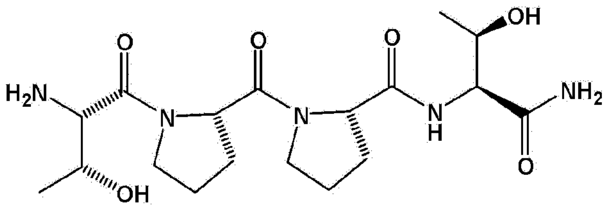

or a pharmaceutically acceptable salt thereof.

In some embodiments, the compositions described herein can have from 1 mL to 4 mL (e.g., 2.5 mL to 3.5 mL,, and preferably 3 mLof an aqueous solution.

The concentration of the GLYX-13 peptide and/or pharmaceutically acceptable salt thereof that is utilized in the compositions described herein may be, for example, depend upon the physiological osmolality that is obtained upon administration. For example, at lower concentrations, e.g. below 100 mg/mL, or below 90 mg/mL or 70mg/mL of the GLYX peptide defined above, the compositions may be hypotonic (lower than physiological osmolality). Hypotonic compositions may lead to adverse patient reactions upon administration, e.g. an injectable hypotonic solution may cause blood cells to expand and break, also known as hemolysis. At higher concentrations, e.g., greater than 200 mg/mL of the GLYX peptide defined above, the compositions (e.g., aqueous solutions) may be hypertonic. Hypertonic compositions may lead to adverse patient reactions upon administration, for example, causing blood cells to shrivel and become crenated.

Following administration of the compositions described herein, a physiological osmolality of from 200 mOsmol/kg to 1000 mOsmol/kg (e.g., from 200 mOsmol/kg to 500 mOsmol/kg, from 500 mOsmol/kg to 1,000 mOsmol/kg, from 600 mOsmol/kg to 950 mOsmol/kg,from 800 mOsmol/kg to 1,000 mOsmol/kg, from 850 mOsmol/kg to 950 mOsmol/kg or 800 mOsmol/kg to 900 mOsmol/kg) is maintained or achieved. The duration of administration is less than 8 hours. In certain embodiments described herein, the duration of injection times range from 1 minute to 5 minutes per dose of composition. In still other embodiments, the duration of injection times are less than 1 minute (e.g., from 1 second to 55 seconds, from 5 seconds to 55 seconds, from 5 seconds to 45 seconds, from 5 seconds to 30 seconds, from 5 second to 15 seconds).

Disclosed hereinare compositions that comprise a GLYX-13 peptide and/or pharmaceutically acceptable salt thereof having a concentration of from 60 mg/mL to 200mg/mL, or 100 mg/mL to 200 mg/mL (e.g., 150 mg/mL or 75 mg/mL). Contemplated compositions for use in treatment include administerating such compositions so upon administration of e.g., 150 mg/ml of the GLYX-13, a physiological osmolality of from 800 mOsmol/kg 900 mOsmol/kg (or 820 to 880 mOsmol/kg), or upon administration of 75 mg/ml of the GLYX-13 a physiological osmolality of from 375 to 475 mOsmol/kg is obtained in said patient.

Disclosed compositions include an acid that provides chloride ions wherein the composition has a pH of 3.9 to 5.5, from 4.0 to 5.0, from 4.2 to 5.0, from 4.2 to 4.8, 4.0, preferably of 4.5 at 25°C. For example, a disclosed composition may include an aqueous solution of GLYX-13 wherein the composition has been adjusted to pH 4.5 (or e.g., 4.1 to about 5.5 at 25 °C) by an acid prepared at 5N, e.g., 5N HCl. Contemplated compositions for use include administerating such compositions for use so upon administration a physiological osmolality of 480 to 960 mOsmol/kg is obtained. For example, contemplated herein are compositions having a GLYX-13 concentration of 120 to 150 mg/mL with osmolalities upon administration, (with a administration duration of e.g. less than 8 hours, less than 4 hours, e.g. 1 minute to 8 hours, or

1 to 5 minutes per dose) of 290 to 360 mOsmol/kg.

Also contemplated herein are compositions that include e..g., a buffer (for example, an acid, for example hydrochloric acid, or chloride ions) and have a pH of from 3.9 to 5.5, from 4.0 to 5.0, from 4.2 to 5.0, from 4.2 to 4.8, preferably of 4.5 at 25°C. The compositions for use comprise administering such a composition to a patient by injection, such that the injection/administration time is from 5 seconds to 5 minutes (e.g., from 5 seconds to 2 minutes, from 5 seconds to 1 minute, from 5 seconds to 55 seconds, from 5 seconds to 45 seconds, from 5 seconds to 30 seconds, from 5 seconds to 15 seconds) and results in a physiological osmolality to between 580 to 720 mOsmol/kg.

Disclosed herein, for example, are compositions that have a GLYX-13 peptide and/or pharmaceutically acceptable salt thereofconcentration of from 100 mg/mL to 200 mg/mL (e.g., 150 mg/mL). Upon administration a physiological osmolality of from 500 mOsmol/kg to 1,000 mOsmol/kg (e.g., from 600 mOsmol/kg to 950 mOsmol/kg, from 800 mOsmol/kg to 1,000 mOsmol/kg, from 850 mOsmol/kg to 950 mOsmol/kg) is obtained.

Disclosed aqueous compositions are stable in that the compound does not rapidly degrade or break down while in solution over time. Thus, in certain embodiments, a disclosed composition has minimal amounts of one or more of degradation products each selected from the group consisting of cyclo-Pro-Thr (proline-threonine-diketopiperazine), Thr-Pro-Pro-Thr, Pro-Pro-Thr, Pro-Pro-Thr-$NH_2$, Thr-Pro, Pro-Thr, Pro-Thr-$NH_2$, proline and/or threonine after 10 days at room temperature, after 20 days at room temperature or after 1 month at 0 °C or below..

The disclosed composition has minimal amounts of one or more of degradation products each selected from the group consisting of cyclo-Pro-Thr, Thr-Pro-Pro-Thr, Pro-Pro-Thr, Pro-Pro-Thr-$NH_2$, Thr-Pro, Pro-Thr, Pro-Thr-$NH_2$, proline and/or threonine after 3 month at from 0°C to 10 °C (e.g., from 2°C to 8 °C, 5 °C), 6 months at from 0°C to 10 °C (e.g., from 2°C to 8 °C, 5 °C), after 9 months at from 0°C to 10 °C (e.g., from 2°C to 8 °C, 5 °C), after 12 months at from 0°C to 10 °C (e.g., from 2°C to 8 °C, 5 °C), after 5 months at from 0°C to 10 °C (e.g., from 2°C to 8 °C, 5 °C), or even after 24 months or more at from 0°C to 10 °C (e.g., from 2°C to 8 °C, 5 °C).

The buffer is selected from the group consisting of acetate, citrate, phosphate, succinic acid, carbonate, bicarbonate, and maleic acid and salts thereof. In another embodiment, the buffer is a salt selected from the group consisting of chloride, sodium, potassium, and ammonium. In yet another embodiment, the GLYX-13 peptide and/or salt thereof having the following formula:

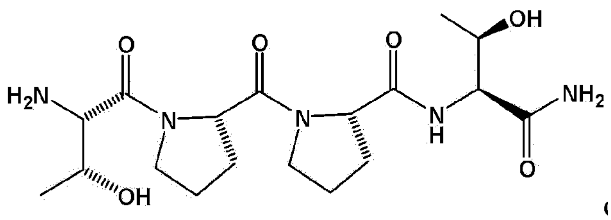

(

or a pharmaceutically acceptable salt thereof) may act as the buffer itself (with, e.g., a pKa of 7 to 7.5). The compositions described herein comprise the GLYX-13 peptide and/or pharmaceutically acceptable salt thereof and an acid (to obtain a desired pH) selected from the group consisting of hydrochloric acid.

The aqueous composition consists essentially of water, the GLYX-13 peptide having the formula shown above or salt thereof and an acid. In some embodiments, the compositions include a cationic counterion that is selected from the group consisting of $H^+$, a protonated form of the peptide, one of more protonated froms of any one or more of the degradation products described herein, or a combination thereof. The cationic counterion is selected from the group consisting of $H^+$ and a protonated form of the peptide, or a combination thereof. In certain embodiments, the compositions are substantially free of cationic sources other than those delineated above, e.g., metal cations, exogenous protonated amino acids or peptides, tetraalkyl ammonium ions, and other protonated acid scavengers.

The disclosed compositions are substantially free of anions other than chloride, e.g., are substantially free of acetate and other carboxylate-containing moieties, bromide, iodide, sulfate-containing moieties, sulfinate-containing moieties, and phosphate-containing moieties.

A cosolvent is present in a disclosed composition and is selected from the group consisting of polyethylene glycol, glycerine, ethanol, polypropylene glycol, and N,N-diemethylacetamide. For example, the cosolvent is polyethylene glycol having a molecular weight of 200 to 900Da. In certain embodiments, the cosolvent is polyethylene glycol having a molecular weight of 400Da, e.g. 400Da to 700Da, .e.g., 200, 300, 400, 500, 500, 700 or 800 Da.

A disclosed composition has minimal amounts of one or more of degradation products each selected from the group consisting of cyclo-Pro-Thr, Thr-Pro-Pro-Thr, Pro-Pro-Thr, Pro-Pro-Thr-$NH_2$, Thr-Pro, Pro-Thr, Pro-Thr-$NH_2$, proline and/or threonine after 1 week, 10 days, 2 weeks, 3 weeks, 4 weeks, 2 months, 3 months, 4 months, 5 months, 6 months, 9 months, 1 year, 2 years, or more at from 2 to 8°C. In another embodiment, a disclosed composition has minimal amounts of one or more of degradation products each selected from the group consisting of cyclo-Pro-Thr, Thr-Pro-Pro-Thr, Pro-Pro-Thr, Pro-Pro-Thr-$NH_2$, Thr-Pro, Pro-Thr, Pro-Thr-$NH_2$, proline and/or threonine after 1 week, 10

days, 2 weeks, 3 weeks, 4 weeks, 2 months, 3 months, 4 months, 5 months, or 6 months, 12 months or more at 25°C.

A disclosed composition has less than 0.5 weight percent (or, e.g, less than 0.7 weight percent, or less than 1 weight percent, less than 2 weight percent, less than 3 weight percent, less than 4 weight percent, , e.g. about 1 to 3 weight percent) of Thr-Pro-Pro-Thr (des amide of GLYX-13) after 3 months, or after 6 months, or after 12 months or more. In another embodiment, the composition has less than 0.1 weight percent Thr-Pro-Pro-Thr after 10 days, or after 20 days, or after 30 days.

A disclosed composition has less than 0.5 or less than 0.6 weight percent (or, e.g, less than about 1.2 weight percent or less than 0.7 weight percent, less than 1 weight percent, less than 2 weight percent, less than 3 weight percent, less than 4 weight percent, less than 5 weight percent, less than 6 weight percent, less than 7 weight percent, where the weight percent of the impurity is a percentage of the pharmaceutically active compound, e.g., GLYX-13) cyclo-Pro-Thr after 3 months, or after 6 months, or after 12 months, 24 months or more at 8°C. A disclosed composition has less than 0.5 weight percent cyclo-Pro-Thr after 10 days, or after 20 days, or after 30 days at 40°C. In still another embodiment, the compositions can include up to 5 weight percent, e.g. 0.01 to 5 weight percent, e.g. 4 to 5 weight percent diketopiperazine upon administration.

A disclosed composition has less than 0.5 weight percent (or, e.g, less than 0.7 weight percent or less than 1 weight percent) Pro-Thr-$NH_2$ after 10 days, or after 20 days, or after 30 days or more. In another embodiment, the composition has less than 0.3 weight percent Pro-Thr-$NH_2$ after 10 days, or after 20 days, or after 30 days at 40°C.

A disclosed composition has less than 0.5 weight percent (or, e.g., less than 0.1 weight percent, or less than 0.4 weight percent or less than 1 weight percent) Thr-Pro-Pro after 10 days, or after 20 days, or after 30 days. In another embodiment, the composition has less than 0.1 weight percent Thr-Pro-Pro after 10 days, or after 20 days, or after 30 days at 40°C.

Disclosed compositions are stable in that the compound does not rapidly degrade or break down while in solution over time. In some embodiments, less than 5% (e.g., less than 4%, less than 3%, less than 2%, less than 1%, less than 0.5%) of the compound rapidly degrades or breaks down while in solution over time (e.g., 10 or more days) and at a temperature that is at, below, or above room temperature.

The disclosure relates to less than 5% (e.g., less than 4%, less than 3%, less than 2%, less than 1%, less than 0.5%) of the compound rapidly degrades or breaks down while in solution after 10 days or more, after 20 days or more, after 30 days or more, after 40 days or more, after 50 days or more, after 60 days or more, or after 90 days or more (e.g., after 20 days or more) at a temperature that is greater than room temperature (e.g., 30 °C, 35 °C , 40 °C, 45 °C; e.g., 40 °C). The disclosure relates to less than 5% (e.g., less than 4%, less than 3%, less than 2%, less than 1%, less than 0.5%) of the compound rapidly degrades or breaks down while in solution after 21 days at 40°C.

The disclosure relates to less than 5% (e.g., less than 4%, less than 3%, less than 2%, less than 1%, less than 0.5%) of the compound rapidly degrades or breaks down while in solution after 10 days or more, after 30 days or more, after 60 days or more, after 90 days or more, after 6 months or more, or after one year or more at room temperature.

Disclosed is less than 5% (e.g., less than 4%, less than 3%, less than 2%, less than 1%, less than 0.5%) of the compound rapidly degrades or breaks down while in solution after 1 month at 0 °C or below, after 3 months at 0 °C or below, after 6 months at 0 °C or below, after 9 months at 0 °C or below, after 12 months at 0 °C or below, after 18 months at 0 °C or below, or after 24 months or more at 0 °C or below.

The compositions described herein can exhibit any two or more of the stability features delineated above.

The disclosed compositions contain minimal amounts of one or both of degradation products diketopiperazine and proline-threonine amide. In other embodiments, the compositions have minimal amounts of one or more degradation products selected from the group consisting of diketopiperazine, proline-threonine amide, and any one or more of the other degradation products described herein.

In certain embodiments, the compositions have less than 1 weight percent (e.g, less than 0.7 weight percent, less than 0.5 weight percent, less than 0.4 weight percent, less than 0.3 weight percent) of diketopiperazine after 10 days or more, after 20 days or more, after 30 days or more, after 40 days or more, after 50 days or more, after 60 days or more, or after 90 days or more (e.g., after 20 days or more) at a temperature that is greater than room temperature (e.g., 30 °C, 35 °C , 40 °C, 45 °C; e.g., 40 °C). In an embodiment, the compositions have less than 0.7 weight percent, less than 0.4 weight percent, or less than 0.3 weight percent of diketopiperazine after 21 days in solution at 40 °C. In still another embodiment, the compositions can include up to 5 weight percent of diketopiperazine upon administration. The weight precent values provided above are determined from the following equation: (weight impurity)/(weight of remaining compound) * 100.

The disclosed compositions have less than 1 weight percent (or, .e.g, less than 0.7 weight percent, less than 0.5 weight percent, less than 0.4 weight percent, less than 0.3 weight percent, less than 0.2 weight percent, or less than 0.1 weight percent) of proline-threonine amide after 10 days or more, after 20 days or more, after 30 days or more, after 40 days or more, after 50 days or more, after 60 days or more, or after 90 days or more (e.g., after 20 days or more) at a temperature that is greater than room temperature (e.g., 30 °C, 35 °C , 40 °C, 45 °C; e.g., 40 °C). In an embodiment, the compositions have less than 0.3 weight percent or less than 0.2 weight percent of proline-threonine amide after 21

days in solution at 40 °C. The weight precent values provided above are determined from the following equation: (weight proline-threonine amide)/(weight of remaining compound) * 100.

In some embodiments, the compositions are prepared by processes that include (i) providing a first combination comprising the compound and water; and (ii) contacting the first combination with hydrochloric acid, or a source thereof, in an amount sufficient to achieve a pH of from 3.9 to 5.5 at 25 °C.

Embodiments can include any one of the features described above as well as any combination of two or more of the features described above.

Medical ConditionsThe intravenous compositions as defined above may be used for the treatment of a variety of other neurological conditions. Exemplary conditions includea learning disorder, autistic disorder, attention-deficit hyperactivity disorder, anxiety, depression, migraine, Tourette's syndrome, phobia, post-traumatic stress disorder, dementia, AIDS dementia, Alzheimer's disease, Parkinson's disease, Huntington's disease, spasticity, myoclonus, muscle spasm, bipolar disorder, neuropathic pain, a substance abuse disorder, urinary incontinence, stroke, ischemia, epilepsy and schizophrenia.

The compositions may be used for treating autism and/or an autism spectrum disorder in a patient need thereof, comprising administering an effective amount of a disclosed composition (e.g. a composition described above) to the patient. The disclosed compositions may be used for reducing the symptoms of autism in a patient in need thereof, comprising administering an effective amount of a disclosed composition to the patient. For example, upon administration, the composition may decrease the incidence of one or more symptoms of autism such as eye contact avoidance, failure to socialize, attention deficit, poor mood, hyperactivity, abnormal sound sensitivity, inappropriate speech, disrupted sleep, and perseveration. Such decreased incidence may be measured relative to the incidence in the untreated individual or an untreated individual(s).

Disclosed are patients suffering from autism also suffer from another medical condition, such as Fragile X syndrome, tuberous sclerosis, congenital rubella syndrome, and untreated phenylketonuria.

The disclosed compositions for use of treating a disorder in a patient in need thereof are contemplated, wherein the disorder is selected from group consisting of: epilepsy, AIDS and/or AIDS dementia, Parkinson's disease, multiple system atrophy, progressive supra-nuclear palsy, Friedrich's ataxia, autism, fragile X syndrome, tuberous sclerosis, attention deficit disorder, olivio-ponto-cerebellar atrophy, cerebral palsy, drug-induced optic neuritis, peripheral neuropathy, myelopathy, ischemic retinopathy, glaucoma, cardiac arrest, behavior disorders, and impulse control disorders that includes administering a disclosed compound, e.g. GLYX-13. Also contemplated herein is a composition for use for treating cough, e.g. uncontrollable cough, comprising administering a GLYX-13 composition to a patient in need thereof.

For example, provided here are compositions for use of treating benign Rolanic epilepsy, frontal lobe epilepsy, infantile spasms, juveline myoclonic epilepsy, Lennox-Gastaut syndrome, Landau-Kleffher syndrome, Dravet syndrome, progressive myoclonus epilepsies, reflex epilepsy, Rasmussen's syndrome, temporal lobe epilepsy, limbic epilepsy, status epilepticus, abdominal epilepsy, massive bilateral myoclonus, catamenial epilepsy, Jacksonian seizure disorder, Lafora disease, and /or photosensitive epilepsy comprising administering an effective amount of a disclosed composition.

Disclosed herein are compositions for use of treating attention deficit disorder, ADHD (attention deficit hyperactivity disorder), schizophrenia (for example, schizo-affective disorders, delusional disorders, e.g., paranoid type, hebephrenic, and/or catatonic type schizophrenia), bipolar disorder (include bipolar I disorder, bipolar II disorder, cyclothymia), borderline personality disorder, anxiety,(include social anxiety disorder, avoidant personality disorder), obsessive-compulsive disorder, amelioration of opiate, nicotine and/or ethanol addiction (e.g., compositions for use of treating such addiction or ameliorating the side effects of withdrawing from such addiction), spinal cord injury diabetic retinopathy, traumatic brain injury, frontal temporal dementia, post-traumatic stress syndrome and/or Huntington's chorea, in a patient in need thereof, that includes administering a disclosed composition. For example, patients suffering from schizophrenia, addiction (e.g. ethanol or opiate), autism (and autism spectrum disorders), Huntington's chorea, traumatic brain injury, spinal cord injury, post-traumatic stress syndrome and diabetic retinopathy may all be suffering from altered NMDA receptor expression or functions.

For example, provided herein is a composition for use of treating schizophrenia, for example, the negative and cognitive symptoms of schizophrenia in a patient suffering therefrom, comprising administering a therapeutically effective amount of a disclosed composition.

Other compositions for use comprise treating stroke and/or ischemia, e.g., ischemic stroke, brain ischemia, transient ischemic attack, cardiac ischemia,and/or myocardial infarction, in a patient in need thereof, comprising administering a pharmaceutically effective amount of a disclosed composition.

Compositions for use comprise modulating an autism target gene expression in a cell comprising contacting a cell with an effective amount of a disclosed composition. The autism gene expression may be for example, selected from ABAT, APOE, CHRNA4, GABRA5,GFAP, GRIN2A, PDYN, and PENK. The compositions for use comprise modulating synaptic plasticity in a patient suffering from a synaptic plasticity related disorder is provided, comprising administering to the patient an effective amount of a disclosed composition.

The compositions for use comprise treating Alzheimer's disease, or e.g., treatment of memory loss that e.g.,

accompanies early stage Alzheimer's disease, in a patient in need thereof is provided, comprising administering a disclosed composition, in particular, modulating an Alzheimer's amyloid protein (e.g., beta amyloid peptide, e.g. the isoform A 1-42), in-vitro or in-vivo (e.g. in a cell) comprising contacting the protein with an effective amount of a disclosed composition. For example, a disclosed composition may block the ability of such amyloid protein to inhibit long-term potentiation in hippocampal slices as well as apoptotic neuronal cell death. A disclosed composition may provide neuroprotective properties to a Alzheimer's patient in need thereof, for example, may provide a therapeutic effect on later stage Alzheimer's-associated neuronal cell death.

Also contemplated herein is a disclosed GLYX-13 composition for use in treatment of clinically relevant antidepressant and anxiolytic and for treatment of depression and anxiety in general.

The disclosure relates at least in part to disclosed GLYX-13 composition alone or in combination with one or more other antidepressant treatments, such as, tricyclic antidepressants, MAO-I's, SSRI's, and double and triple uptake inhibitors and/or anxiolytic drugs for use in treating depression, anxiety, and/or other related diseases including providing relief from depression, anxiety and preventing recurrence of depression and anxiety. Exemplary drugs that may be used in combination with a GLYX peptide include Anafranil, Adapin, Aventyl, Elavil, Norpramin, Pamelor, Pertofrane, Sinequan, Surmontil, Tofranil, Vivactil, Parnate, Nardil, Marplan, Celexa, Lexapro, Luvox, Paxil, Prozac, Zoloft, Wellbutrin, Effexor, Remeron, Cymbalta, Desyrel (trazodone), and Ludiomill. It will be appreciated that in some embodiments, administration of a disclosed GLYX-13 composition may act more quickly than a co-administered antidepressant treatment, and thus such coadministration (e.g., administration of GLYX-13 on an acute or immediate basis, while starting a regimen with another, slower acting anti-depressant at about the same time) may be particularly advantageous in the common situation where the second antidepressant is slower acting.

Disclosed is the stable, aqueous composition as described above for use in treating depression that includes administering a disclosed composition in combination with (e.g. simultaneously or sequentially) other non-pharmacological treatments such as psychotherapy, electroconvulsive therapy, vagus nerve stimulation and/or transcranial magnetic stimulation.

A variety of depression conditions are expected to be treated according to this aspect of the disclosure without affecting behavior or motor coordination, and without inducing or promoting seizure activity. Exemplary depression conditions that are expected to be treated according to this aspect of the disclosure includemajor depressive disorder, dysthymic disorder, psychotic depression, postpartum depression, premenstrual syndrome, premenstrual dysphoric disorder, seasonal affective disorder (SAD), anxiety, mood disorder, depressions caused by chronic medical conditions such as cancer or chronic pain, chemotherapy, chronic stress, post-traumatic stress disorders, risk of suicide, and bipolar disorder (or manic depressive disorder). It should be understood that depression caused by bipolar disorder may be referred to as bipolar depression. In addition, patients suffering from any form of depression often experience anxiety. Various symptoms associated with anxiety include fear, panic, heart palpitations, shortness of breath, fatigue, nausea, and headaches among others. It is expected that the described compositions can be used to treat anxiety or any of the symptoms thereof.

The compositions disclosed may equally be used for treating depression in treatment resistant patients or treating refractory depression, e.g., patients suffering from a depression disorder that does not, and/or has not, responded to adequate courses of at least one, or at least two, other antidepressant compounds or therapeutics. For example, provided herein is a method of treating depression in a treatment resistant patient, comprising a) optionally identifying the patient as treatment resistant and b) administering an effective dose of a disclosed GLYX-13 composition to said patient.

Provided herein, in an embodiment is a composition as defined above for use of acutely treating symptoms of depression in a patient in need thereof, comprising administering an effective amount of GLYX-13, for example, in a single unit dose intravenously. Such treatment may relieve the patient of at least one symptom of depression for about 2 weeks or less, 1 week or less, 1 day or less, or 1 hour or less (e.g. 15 minutes or less, half an hour or less), after said administration or may relieve the patient of at least one symptom of depression for about 1 day or more, 1 week or more, or 2 weeks or more after said administration. For example, provided herein is a method comprising administering an effective amount of GLYX-13 to a patient suffering from depression, wherein said patient is substantially relieved of at least one symptom of depression substantially earlier after the first administration of GLYX-13, as compared to the same patient administered a non-GLYX-13 antidepressant compound. One of skill in the art will appreciate that such methods of acute administration may be advantageous in a hospital or out-patient setting.

Symptoms of depression, and relief of same, may be ascertained by a physician or psychologist, e.g., by a mental state examination. Symptoms include thoughts of hopelessness, self-harm or suicide and/or an absence of positive thoughts or plans.

The patient is a human, e.g. a human pediatric patient.

In some embodiments, a disclosed composition alone or in combination with one or more other agents is used for treating depression or another contemplated indication.

GLYX-13 may provide a high therapeutic index. For example, GLYX-13 may be therapeutically effective with

an i.v. dose range of about 1 to 10 mg/kg. In some embodiments, no ataxia occurs, at for example a dose of at 500 mg/kg, i.v.

The administration may include one dose, or one or more doses, of a disclosed composition. In some embodiments, a patient has substantial improvement after 12 hours, after 1 day, after 1 week, after 2 days, after 3 days, after 4 days, after 5 days, after 6 days, or even after 8 days of a one (single) dose administration.

A therapeutically effective amount of a disclosed composition required for use in therapy varies with the nature of the condition being treated, the length of treatment time desired, the age and the condition of the patient, and is ultimately determined by the attending physician. In general, however, dosage forms employed for adult human treatment typically are in the range of 60 mg/mlto 200 mg/ml. In some embodiments, disclosed dosage forms are capable of delivering about 0.5 to 2 grams per day of GLYX-13 to a patient. A number of factors may lead to the disclosed composition being administered over a wide range of dosages. When given in combination with other therapeutic agents, the dosage of the disclosed compositions may be given at relatively lower dosages. As a result, the dosage form of a disclosed composition may be from 10 mg/ml to 70 mg/ml, or for example, 70 mg/mL to 200 mg/ml. The dosage of a disclosed composition may be at any dosage including, but not limited to, 10 mg/ml, 15 mg/ml, 20 mg/ml, 25 mg/ml, 30 mg/ml, 35 mg/ml, 40 mg/ml, 45 mg/ml, 50 mgl/ml, 55 mg/ml, 60 mg/ml, 65 mg/ml, or 70 mg/ml. More concentrated solutions, including 80 mgl/ml, 100 mg/ml, 125 mg/ml, 150 mg/ml, or 200 mg/ml are also disclosed as convenient dosage forms. The desired dose may be conveniently administered in a single dose, or as multiple doses administered at appropriate intervals, for example as two, three, four or more sub-doses per day. Pharmaceutical compositions of the disclosure suitable for parenteral administration comprise a subject composition in combination with one or more pharmaceutically-acceptable sterile isotonic aqueous or non-aqueous solutions, dispersions, suspensions or emulsions, or sterile powders which may be reconstituted into sterile injectable solutions or dispersions just prior to use, which may contain antioxidants, buffers, bacteriostats, solutes which render the formulation isotonic with the blood of the intended recipient or suspending or thickening agents.

Examples of suitable aqueous and non-aqueous carriers which may be employed in the pharmaceutical compositions of the disclosure include water, ethanol, polyols (such as glycerol, propylene glycol, polyethylene glycol, and the like), and suitable mixtures thereof, vegetable oils, such as olive oil, and injectable organic esters, such as ethyl oleate and cyclodextrins. Proper fluidity may be maintained, for example, by the use of coating materials, such as lecithin, by the maintenance of the required particle size in the case of dispersions, and by the use of surfactants.

The invention now being generally described, it will be more readily understood by reference to the following examples.

**EXAMPLE 1 GLYX-13, 60mg/mL in saline solution, pH 6.5 to 7.0**

Prepare 90 g/L of sodium chloride solution in water for injection USP (WFI).

Preparation of 180 mg/ml GLYX-13 Solution

Add water for injection USP (WFI) to a compounding vessel, to an amount equivalent to 20% of the final QS weight and record the exact weight added. Begin mixing the solution and add calculated amount of Acetic Acid USP to the compounding vessel. The amount of acetic acid is 0.9 to 1.0 molar equivalent of the target amount of GLYX-13 in the formulation batch.

Add the calculated amount of GLYX-13 free base to the compounding vessel and record the exact weight added. Continue mixing for 15 to 30 minutes.

Stop mixing and add calculated amount of 90 g/L saline solution to the compounding vessel. The amount of NaCl must be equal to the amount needed reach 9 g/L in the final formulation.

Preparation of 60 mg/ml GLYX-13 Solution

Transfer the entire content of the compounding vessel into a polymeric mixing bag or large stainless steel mixing vessel. Mix for 15-30 minutes. Add sufficient amount of WFI to the solution to reach the final target final volume. Test the content of GLYX-13 by HPLC and test the pH. If the pH is outside the range of 6.6 to 6.9, adjust the pH with acetic acid or NaOH. The final batch will contain, per liter of solution, 60 grams GLYX-13 and 6 to 6.4 grams Acetic Acid in 0.9 % sodium chloride; the pH must be between 6.5 and 7.0.

Sterilization and Vial Filling

The solution is sterilized by aseptic filtration through the following three filters in series: a pre-filtration with 0.45 μm filter, followed by two 0.22 μm filters. Fill each vial with 20ml (20 to 20.5 mL) of the bulk sterile solution, stopper and cap vials. Each vial contains 1.2 grams of GLYX-13 in 20 mL solution.

**EXAMPLE 2 GLYX-13 in Buffer Solutions**

A. Prepare 0.1 M Tris Buffer Solution ("TrisHCl-7.0") in water, pH 7.0 using HCl to adjust the pH.

Preparation of 60 mg/ml GLYX-13 Solution

Add approximately 75% the target volume of TrisHCl-7.0 buffer to a compounding vessel. For each liter of the target batch volume, weigh 60 g of GLYX-13 free base and add to the compounding vessel. Mix for 15 min. Measure the pH. If pH is out of range (7.0±0.05), adjust accordingly with HCl or NaOH. Add enough TrisHCl-7.0 to reach the final batch volume. The final bulk solution will contain, per liter of solution, 60 grams GLYX-13 free base in 0.1 M Tris(hydroxymethyl)aminomethane/HCl buffer, pH 7.0.

Sterilization and Vial Filling

Filter sterilize and fill the vials as described above. Each vial contains 1.2 grams of GLYX-13 in 20 mL solution (GLYX-13 60 mg/mL, tris(hydroxymethyl)aminomethane/HCl buffer, pH 7.0).

B. 60 mg/mL in Citrate Buffer solution, pH 5.6: Prepare 0.1 M trisodium citric acid solution in water and adjust the pH to 5.6 with HCl and NaOH as needed.

Preparation of 60 mg/ml GLYX-13 Solution

Add approximately 75% the target batch volume of citric acid/sodium citrate buffer, pH 5.6 to a compounding vessel. For each liter of the target batch volume, weigh 60 g of GLYX-13 free base and add to the compounding vessel. Mix for 15 min. Measure the pH. If pH is out of range (5.6±0.05), adjust accordingly with HCl or NaOH. Add enough citric acid/sodium citrate buffer (pH 5.6) to reach the final batch volume. The final bulk solution will contain, per liter of solution, 60 grams GLYX-13 free base in 0.1 M citric acid/sodium citrate buffer, pH 5.6.

C. Sterilization and Vial Filling

Filter sterilize and fill the vials as described above. Each vial contains 1.2 grams of GLYX-13 in 20 mL solution. (GLYX-13 in citric acid/sodium citrate buffer, pH 5.6)

C. GLYX-13, 200 mg/mL in Tris Buffer solution, pH 7.5

A. Prepare 0.1 M Tris Buffer Solution ("TrisHCl-7.5") in water, pH 7.5 using HCl to adjust the pH.

B. Preparation of 200 mg/ml GLYX-13 Solution

Add approximately 50% the target volume of TrisHCl-7.5 buffer to a compounding vessel. For each liter of the target batch volume, weigh 200 g of GLYX-13 free base and add to the compounding vessel. Mix for 15 min. Measure the pH. If pH is out of range (7.5±0.05), adjust accordingly with HCl or NaOH. Add enough TrisHCl-7.5 to reach the final batch volume. The final bulk solution will contain, per liter of solution, 200 grams GLYX-13 free base in 0.1 M Tris(hydroxymethyl)aminomethane/HCl buffer, pH 7.5.

Sterilization and Vial Filling

Filter sterilize and fill the vials as described above. Fill each vial with 5 ml (4.85 to 5.15 mL) of the bulk sterile solution, stopper and cap vials. Each vial contains 1 grams of GLYX-13 in 10 mL solution. (GLYX-13 200 mg/mL, Tris(hydroxymethyl)aminomethane/HCl buffer, pH 7.5)

D. GLYX-13, 200 mg/mL in Tris Buffer and 40% PEG400 solution, pH 7.5

. Prepare 0.1 M Tris Buffer Solution ("TrisHCl-7.5") in water, pH 7.5 using HCl to adjust the pH.

Preparation of 200 mg/ml GLYX-13 Solution

Add approximately 20 L of 0.2 M TrisHCl-7.5 buffer to a compounding vessel. Add 40 L of PEG400 to the vessel and stir for 30 min. Weigh 20 kg of GLYX-13 free base and add to the compounding vessel. Mix for 0.5 to 2 hours.

Remove a sample and measure the pH. If pH is out of range (7.5±0.05), adjust accordingly with HCl or NaOH. Add enough TrisHCl-7.5 (approximately 30 L) to reach the final batch volume of 100 L. The final bulk solution will contain, per liter of solution, 200 grams GLYX-13 free base and 40% PEG400 in 0.1 M Tris(hydroxymethyl)aminomethane/HCl buffer, pH 7.5.

Sterilization and Vial Filling

Filter sterilize and fill the vials as described above. Fill each vial with 10 ml (10 to 10.5 mL) of the bulk sterile solution, stopper and cap vials. Each vial contains 2 grams of GLYX-13 in 10 mL solution. (GLYX-13 200 mg/mL, in 40 % Polyethylene glycol 400 (PEG400), Tris(hydroxymethyl)aminomethane/HCl buffer, pH 7.5)

**EXAMPLE 3 Stability of GLYX-13 I.V. Solutions**

The stability of GLYX-13 is tested by HPLC using the conditions below.

HPLC Analytical Method

Column: RP-C18 ODS 2, (5 μm, 80A, 4.6 x 250 mm)

Eluent A: 13.4 g potassium dihydrogen phosphate and 4.4 g 1-heptanesulfonic acid sodium salt in 1600 mL water. Adjust pH to 2.5 with o-phosphoric acid. Dilute to 2 L with water.
Eluent B: 1400 mL Methanol and 600 mL Water.

Gradient: 20% B to 70% B in 25 min.; 70% B to 20% B in 3 min.; 0% B for 8 min.
Flow Rate: 1 mL/min.
Detection: UV (220 nm)
Injection: 20 μL of 1 mg/mL solution
Temperature: Ambient

The retention time for GLYX-13 is 12.0 to 12.8 minutes. Thr-Pro-Pro-Thr (TPPT = deaminated GLYX-13) has a retention time of 14.9 to 15.1 minutes. Thr-Pro-Pro (TPP) has retention time of 4.8 to 5.2 minutes. Impurity peaks using this method are analyzed in terms of percent (%) area, versus the GLYX-13 peak and not presented by weight/weight percentage.

**EXAMPLE 4 Low pH Formulations**

GLYX-13 was formulated at 150 mg/mL and adjusted to specific pH with either acetic acid or hydrochloric acid. The formulations were then subjected to accelerated stability conditions (2 months at 40° C). Table 1 provides a summary of the key stability-limiting attributes versus pH and counter ions (acetic acid and HCl).

**Table 1. Stability of GLYX-13 Solutions at 40° C for 2 months.**

| pH<br>Counter ion | AA-5<br>5.0<br>Acetic Acid | HCl-5<br>5.0<br>HCl | HCl-4<br>4.0<br>HCl | HCl-8<br>8.0<br>HCl |
|---|---|---|---|---|
| Assay: % Label Claim | 62.0 | 89.3 | 90.8 | 76.5 |
| Impurity 1. RRT 0.43 (% area) | 16.0 | 0.97 | 0.69 | 1.87 |
| Impurity 2. RRT 0.57 (% area) | 4.4 | 0.34 | 0.25 | 0.54 |
| Total Impurities[1] (% area) | 22.37 | 2.54 | 2.22 | 13.50 |
| 1.At the beginning of the study, the % claim was 93%, and the total impurity levels were 1.6%. Impurities are reported as % of the GLYX-13 peak from the sample. Total impurities includes total of all individual impurities at levels greater than 0.05%. Impurities 1 and 2 represent the two major impurities that increase over time. The rate of impurity formation is dependent on counter ion, pH, and temperature. | | | | |

Table 1 indicates that HCl as the counter ion stabilizes the formulation relative to acetic acid. At pH 5.0 the levels of impurities are 10-fold lower and the % label claim (150 mg) of GLYX-13 is almost 50% higher in HCl solution

versus acetic acid solution (see Forms AA-5 versus HCl-5). Further, lower pH solutions are more stable than high-pH solutions: note that in HCl solutions, the levels of impurities are significantly lower and the % label claim (150 mg) of GLYX-13 are significantly higher at low pH (4 and 5) versus higher pH (8) solution (see Formulations HCl-4 and HCl-5 versus Form HCl-8).

**EXAMPLE 5 Syringe/Container Stability Study**

The objective of this study was to examine the stability of the GLYX-13 150 mg/mL formulation filled into syringes and vials. Four different syringe and stopper combinations were examined along with one vial and stopper configuration.

**Table 2**

| Container | Closure |
|---|---|
| BD glass syringes | HyPak SCF plunger |
| BD glass syringes | Sterifill plunger |
| Schott TopPac syringes | FM257 Plunger |
| Schott TopPac syringes | Stelmi Plunger |
| Schott glass vial | 13 mm stopper |

A Flexicon peristaltic pump equipped with Flexicon tubing was calibrated to deliver specific fill volumes based on the density of the drug solution. The density of the drug solution at 25°C was 1.044 g/mL. The glass syringes were filled with 5.1 mL or 5.32 g of solution and the polymeric syringes were filled with 5.14 mL or 5.37 g of solution. Extra solution was included in each syringe to account for volume retained by the syringe / stopper components that is unavailable for delivery. Each vial was filled with 5.32 g of solution to account for the volume retained by the vial.

The stability of the drug solution was compared with a placebo solution filled into the same container/closure combinations. Samples were stored at -20°C, 2-8°C, 25°C, and 40°C for the durations listed in the Tables 3 - 6.

Testing Key for the tables:

Samples containing active at 150 mg/mL:

A = pH, appearance, assay / related substances, color, deliverable volume (1 syringe required for all testing per time point.)

B = Deliverable volume, HIAC, osmolality, and optical rotation (5 syringes needed per time point.)

Samples containing placebo:

A = pH, appearance, assay / related substances, color (1 syringe required per time point.)

B = Deliverable volume, HIAC (5 syringes required per time point.)

**Table 3:** Time Points and Testing for Samples Stored at -20 °C

| Time Points | Testing |
|---|---|
| T0 | A, B |
| 12 weeks | A, B |

**Table 4: Time Points and Testing for Samples Stored at 2-8 °C**

| Time Points | Testing |
|---|---|
| T0 | A, B |
| 4 weeks | A |
| 8 weeks | A, B |
| 12 weeks | A, B |
| 24 weeks | A, B |

**Table 5: Time Points and Testing for Samples Stored at 25 °C**

| Time Points | Testing |
|---|---|
| T0 | A, B |
| 4 weeks | A |
| 8 weeks | A, B |
| 12 weeks | A, B |
| 24 weeks | A, B |

**Table 6: Time Points and Testing for Samples Stored at 40 °C**

| Time Points | Testing |
|---|---|
| T0 | A, B |
| 2 weeks | A |
| 4 weeks | A |
| 6 weeks | A |
| 8 weeks | A, B |
| 12 weeks | A, B |

The testing and specifications for the syringes a Samples are tested for the following a selected time points. Not all tests are performed at each timepoint.: pH, appearance, assay / related substances, color, deliverable volume, particle size distribution (HIAC), osmolality, and optical rotation.

**Table 3:** Time Points and Testing for Samples Stored at -20 °C

| Time Points |
|---|
| Initial (T0) |
| 12 weeks |

**Table 4: Time Points and Testing for Samples Stored at 2-8 °C**

| Time Points |
|---|
| Initial (T0) |
| 4 weeks |
| 8 weeks |
| 12 weeks |

(continued)

| Time Points |
|---|
| 24 weeks |

**Table 5: Time Points and Testing for Samples Stored at 25 °C**

| Time Points |
|---|
| Initial (T0) |
| 4 weeks |
| 8 weeks |
| 12 weeks |
| 24 weeks |

**Table 6: Time Points and Testing for Samples Stored at 40 °C**

| Time Points |
|---|
| Initial (T0) |
| 2 weeks |
| 4 weeks |
| 6 weeks |
| 8 weeks |
| 12 weeks |

The testing and speficiations for the syringes and vials in the stability study are listed in Table 7. Testing was performed initially and after 4, 8, 12, and 24 weeks. Samples from the 2 week time point were frozen and tested at the same time as the 4 week samples. The samples from the 6 week time point were frozen and tested at the same time as the 8 week samples.

The syringes were stored in bags so that they remained horizontal during storage. Approximately 65 vials were stored in the upright position and 55 vials were stored inverted. The inverted samples were removed after 4, 8, 12, and 24 weeks of storage at each condition. Extra syringes were included per time point.

**Table 7: Testing and Specifications for the Drug Solution**

| Attribute | Test Method | Acceptance Criteria |
|---|---|---|
| Appearance | TBD | Clear solution, colorless to faint yellow, essentially free of visible contamination |
| UV Absorbance at TBD $cm^{-1}$ | TBD | Report Result |
| Optical rotation | TBD | Report Result |
| Osmolality | USP<785> | Report Result |
| Identification | HPLC | Retention time of the sample corresponds to the retention time of the standard |
| Assay | HPLC | 90.0-110.0 %LC |

(continued)

| Attribute | Test Method | Acceptance Criteria |
|---|---|---|
| Chromatographic Purity, Related Substances (%Area) | HPLC | (report any individual ≥0.05%)<br>Individual unspecified impurities: ≤0.5%<br>Total Unspecified: NMT 2.0%<br>Specified Unidentified impurities:<br>NRX-2181 (Thr-Pro-Pro-Thr), NMT 0.5%<br>NRX-1160 (Cyclo-Pro-Thr) , NMT 2.0%.<br>NRX-1152 (Pro-Thr-NH2), NMT 1.0%.<br>NRX-1161 (Pro-Thr), NMT 2.0%.<br>Total impurities: ≤5.0% |
| pH | USP <791> | GLYX-13 4.5 - 5.0 |
| Particulate Matter | USP <788> small volume injections Method 2 | ≥10μm NMT 6000 particles<br>≥25μm NMT 600 particles |
| Volume per Container | USP <1> | Not less than 5 mL |

There was no significant difference in syringe container/closure configurations. All container/closure configurations met the acceptance criteria for appearance, identity, assay, specified related substances, total related substances, pH (active), particulate matter (active), and volume per container. Individual unspecified related substances and total unspecified related substances after 4 and 8 weeks for all container/closure configurations did not meet acceptance criteria in some instances.

**EXAMPLE 6 Stability Study**

Test and Control Articles

GLYX-13 Drug substance; and 5 M hydrochloric acid solutions; Methanol; 10 mL vial with 20 mm opening; 20 mm Daikyo solution stopper; Syringe filters, 0.2μm Millex-GV

HPLC Method

| | |
|---|---|
| Column: | Waters Spherisorb ODS2, 3 μm, 4.6 x 250 mm, Part # PSS832115 |
| Mobile phase A: | 2.2 g/L heptanesulfonic acid, 6.7 g/L potassium dihydrogen phosphate, pH 2.5 |
| Mobile phase B: | 70/30 Methanol/Water |
| Needle wash: | water |
| Column temperature: | 40 ± 1 °C |
| Sample temperature: | 4 ± 1 °C |
| Wavelength: | 210 nm |
| Run time: | 50 min |
| Flow rate: | 1.0 mL/min |

Gradient:

| Time | %B |
|---|---|
| 0 | 0 |
| 35 | 50 |
| 40 | 70 |
| 41 | 00 |
| 50 | |

Study

Four formulations were prepared to examine dissolution of the drug based (Table 2). Each formulation was prepared at 35 mL and contained 5.25 g of GLYX-13.

**Table 8. Formulations for the pH Study**

| Formulation | GLYX-13 (mg/mL) | pH | pH Adjustment Acid |
|---|---|---|---|
| 1 | 150 | 4 | HCl |
| 2 | 150 | 5 | HCl |
| 3 | 150 | 6 | HCl |
| 4 | 150 | 7 | HCl |

Each formulation was prepared by adding the drug to a beaker and adjusting the volume to approximately 26 mL using purified water. The pH was adjusted using 5 M HCl that was added in small increments. The solution was mixed for 10 minutes after each adjustment and mixed for 30 minutes after reaching the desired pH. The pH of the solution was checked after 30 minutes and adjusted if needed. The solution was adjusted to 35 mL using purified water when a drift in pH was no longer observed. Each formulation was filled into vials (2.5 mL/vial), sealed with a stopper, and capped. The formulations were stored at 40°C for 3 weeks and samples were removed weekly. All weekly samples were examined for appearance, tested for pH, assay and related substances using the HPLC assay.

**Table 9. Stability of GLYX-13 Solutions at 40° C for 3 weeks.**

| **Form Code**<br>**pH**<br>**Counter ion** | **HCL-4**<br>**4.0**<br>**HCl** | **HCL-5**<br>**5.0**<br>**HCl** | **HCL-6**<br>**6.0**<br>**HCl** | **HCL-7**<br>**7.0**<br>**HCl** |
|---|---|---|---|---|
| Assay: % Label Claim | 102 | 102 | 101 | 96 |
| Impurity 1. RRT 0.43 (%) | 0.29 | 0.34 | 0.63 | 1.87 |
| Impurity 2. RRT 0.57 (%) | 0.13 | 0.16 | 0.25 | 0.68 |
| Total Impurities[1] (%) | 1.62 | 1.67 | 2.09 | 4.57 |

1. At the beginning of the study, the % label claim was 104%, and the total impurity levels were 1.2%. Impurities are reported as % area of the GLYX-13 peak from the sample. Total impurities includes total of all individual impurities at levels greater than 0.05%. Impurities 1 and 2 represent the two major impurities that increase over time.

Data were analyzed using the appropriate software designed for the assay and impurities HPLC method. Table 3 provides a summary of the stability data at the 3-weeks testing point. Impurity 1 (RRT 0.43) is cyclo-proline-threonine ("diketopiperazine"). Impurity 2 (RRT 0.57) is proline-threonine amide. Figure 1 shows the formation of impurity 1 (RRT 0.43) over time versus pH. Figure 2 shows the formation of Impurity 2 (RRT 0.57) over time versus pH. All data is reported as % area of the GLYX-13 peak - not on weight/weight%).

The above examples indicate that the solutions maintained at low pH and neutral pH are more stable than those maintained at high pH. For example, the % (150 mg) of GLYX-13 is significantly higher at low pH (4 and 5) versus higher pH (7) solution (see Forms HCl-4 and HCl-5 versus Form HCl-7); the levels of the two specified impurities increase significantly with increasing of pH and the level of total impurities of GLYX-13 solutions at low pH stored at 40° C for 3 weeks is 3-fold lower at pH 4 relative to pH 7 solution.

The results show that the stability of GLYX-13 solution is inversely proportional to the pH. The rate of impurity formation at pH 7 is approximately 6-fold greater than the rate at pH 4. Such improved stability of a peptide in HCl at low pH e.g. about 4 to about 5, relative to neutral pH (6-8, e.g. 7) is surprising, in part because peptides are typically subject to hydrolysis at low pH in HCl. In addition, the decrease in GLYX-13 concentration/increase in GLYX-13 degradation products observed during stability testing were less for formulations prepared with HCl than with those prepared with acetic acid.

Example 6 Acid Study

The purpose of this study is to test the effect of the type of acid used for pH adjustment on the stability of

rapastinel (GLYX-13) 150 mg/ml.

Rapastinel 150 mg/ml solution is prepared with rapastinel powder dissolved in water and the pH is adjusted to 4.5 using 5N HCl. This protocol describes the preparation of rapastinel 150 mg/ml solution with a pH adjust to 4.5 using 10 different types of acid all prepared at 5N - except as noted below.

Table 10. Materials, Suppliers for formulations for testing.

| Material | Supplier |
|---|---|
| Purified Water | Milli-Q |
| Fumaric Acid | Acros |
| Malic Acid | Acros |
| Lactic Acid | Sigma Aldrich |
| 5N HCl | Baxter |
| Acetic Acid | EMD |
| Citric Acid | Sigma Aldrich |
| Phosphoric Acid | EMD |
| Nitric Acid | JT Baker |
| Sulfuric Acid | Sigma |
| Ascorbic Acid | JT Baker |

Table 11. Equipment List.

| Equipment | Supplier |
|---|---|
| Orion pH Meter Model 920A | Thermo Scientific |
| Vacuum Filtration System 0.2 micrometer PES PES Filter | VWR |

I. Acid Preparation

A. 5N HCl Solution This study will use 5N HCl solution prepared previously.

B. Fumaric Acid Solution

$$\text{Molecular weight of fumaric acid} = 116.07 \text{ x } 5\text{N} = 580.35 \text{ mg/mL x } 250 \text{ mL} = 145087.5 \text{ mg.}$$

Fumaric acid has low solubility in water, so the solution is prepared near its maximum solubility and concentrated solutions of rapastinel (450 mg/mL) is used to facilitate pH adjustment.

C. Malic Acid Solution

$$\text{Molecular weight of malic acid} = 134.09 \text{ x } 5\text{N} = 670.45 \text{ mg/mL x } 250 \text{ mL} = 167612.5 \text{ mg}$$

$$\text{Molecular weight of malic acid} = 116.07 \text{ x } 5\text{N} = 580.35 \text{ mg/mL x } 250 \text{ mL} = 145087.5 \text{ mg.}$$

Malic acid has low solubility in water, so the solution is prepared near its maximum solubility and concentrated solutions of rapastinel (450 mg/mL) is used to facilitate pH adjustment.

D. Lactic Acid Solution

The solution is prepared near its maximum solubility and concentrated solutions of rapastinel (450 mg/mL) is used to facilitate pH adjustment.

E. 5N Acetic Acid Solution (250 mL)

$$\text{Molecular weight of acetic acid} = 60.05 \text{ x } 5\text{N} = 300.25 \text{ x } 250 \text{ mL} = 75062.5 \text{ mg}$$

1. Add 75.06 g acetic acid to a 250 ml volumetric flask.
2. QS to 250 mL using purified water and mix thoroughly.

F. 5N Citric Acid Solution (250 mL)

$$\text{Molecular weight of citric acid} = 192.12 \text{ x } 5\text{N} = 960.6 \text{ x } 250 \text{ mL} = 240150 \text{ mg}$$

1. Add 240.15 g citric acid to a 250 volumetric flask.
2. QS to 250 mL using purified water and mix thoroughly.

G. 5N Phosphoric Acid Solution (250 ml)

$$\text{Molecular weight of phosphoric acid} = 98 \text{ x } 5\text{N} = 490 \text{ x } 250 \text{ ml} = 122500 \text{ mg}$$

1. Add 122.5 g phosphoric acid to a 250 ml volumetric flask.
2. QS to 250 ml using purified water and mix thoroughly.

H. 5N Nitric Acid Solution (250 ml)

$$\text{Molecular weight of nitric acid} = 63.01 \text{ x } 5\text{N} = 315.05 \text{ x } 250 \text{ ml} = 78762.5 \text{ mg}$$

1. Add 78.76 g nitric acid to a 250 ml volumetric flask.
2. QS to 250 ml using purified water and mix thoroughly.

I. 5N Sulfuric Acid Solution (250 ml)

$$\text{Molecular weight of sulfuric acid} = 98.08 \text{ x } 5\text{N} = 490.4 \text{ x } 250 \text{ ml} = 122600 \text{ mg}$$

1. Add 122.6 g sulfuric acid to a 250 ml volumetric flask.
2. QS to 250 ml using purified water and mix thoroughly.

J. 5N Ascorbic Acid Solution (250 ml)

$$\text{Molecular weight of ascorbic acid} = 176.12 \text{ x } 5\text{N} = 880.6 \text{ x } 250 \text{ ml} = 220150 \text{ mg}$$

1. Add 220.15 g ascorbic acid to a 250 ml volumetric flask.
2. QS to 250 ml using purified water and mix thoroughly.

II. Rapastinel 150 mg/ml Preparation

Rapastinel, 150 mg/ml, solutions will be prepared at 250 ml each. The theoretical quantity of rapastinel needed is 37.5 g per 250 ml solution. The actual weight of rapastinel will be adjusted based on potency, residual moisture, total impurities, residualsolvents, and residue on ignition (Table 12).

**Table 12. Data for Adjustment of Total Quantity of Rapastinel.**

| Total Impurities | Water Content(%) | Residual Solvents | Residue on Ignition | Potency(%) |
|---|---|---|---|---|
| 1.52 | 3.3 | 0.3632 | NA | 99.1 |

100 + 1.52+3.3+0.3632 = 105.18 37.5 g x 1.0518 = 39.44 g; Adjust for potency: 100- 99.1 = 0.9; 39.44 x 1.009 = 39.79 g Rapastinel needed per 250 ml batch.

1. Prepare 10 different Rapastinel, 150 mg/ml solutions by dissolving 39.79 g of Rapastinel in approximately 180 ml purified water.
2. Test and record the pH of the drug solution.
3. Adjust the pH of each solution to 4.5 using a different acid for each batch.

    a. Add the acid incrementally and record the volume of acid added at each increment.
    b. Record the pH of the solution after each addition of acid.

4. Mix the solution for 1 hour after reaching pH 4.5.
5. Test and record the pH after 1 hour.
6. Adjust the pH to 4.5 if needed using the respective acid for the solution. Record the volume of acid added and the final pH.
7. QS the solution to 250 ml using purified water, mix well, and record the pH.
8. Filter each solution through a vacuum filtration system with 0.2 1-lm filter.
9. Submit one sample of each solution as T=0 samples for HPLC assays.
10. Store each solution at 40°C and remove one sample from each batch after storage for 2 weeks and 4 weeks. Freeze the 2 week and 4 week samples until they can be tested together.
11. Samples are tested for pH, appearance, assay / related substances, color, deliverable volume, particle size distribution (HIAC), osmolality, and optical rotation.

EQUIVALENTS

**Claims**

**1.** A stable, aqueous composition suitable for intravenous injection, comprising:
60 mg/mL to 200 mg/mL of a pharmaceutically active compound having the formula:

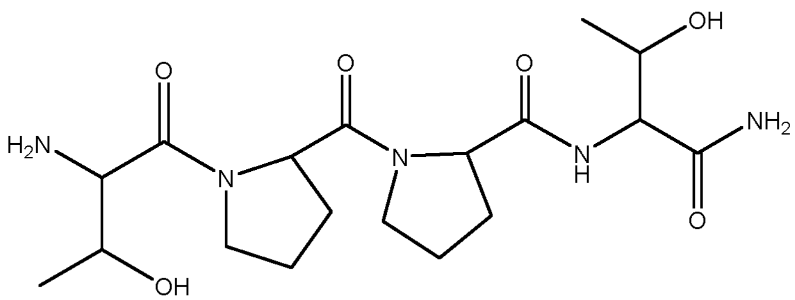
;

or a pharmaceutically acceptable salt thereof;
water for injection; and
an acid; wherein the stable, aqueous composition has a pH of from 3.9 to 5.5 at 25 °C; and
wherein the acid provides chloride ions in the aqueous composition.

**2.** The stable, aqueous composition of claim 1, comprising from 75 mg/mL, 150 mg/mL, or from 125 mg/mL to 175 mg/mL of the pharmaceutically active compound.

3. The stable, aqueous composition of any one of claims 1-2, comprising 200 mg to 500 mg of the pharmaceutically active compound, preferably 225 mg, 375 mg or 450 mg of the pharmaceutically active compound.

4. The stable, aqueous composition of any one of claims 1-3, wherein the stable, aqueous composition has a pH of 4.5 at 25 °C.

5. The stable, aqueous composition of any one of claims 1-4 comprising at least one of: $H^+$, a protonated form of the pharmaceutically active compound, and/or a combination thereof.

6. The stable, aqueous composition of any one of claims 1-5, wherein upon administration of a dose of the stable, aqueous liquid composition that comprises 150 mg/mL of the pharmaceutically active compound and has a volume of 3 mL to a patient, a physiological osmolality of from 800 mOsmol/kg to 900 mOsmol/kg is obtained in said patient.

7. The stable, aqueous composition of any one of claims 1-5, upon administration of a dose of the stable, aqueous liquid composition that comprises 75 mg/mL of the pharmaceutically active compound and has a volume of 3 mL to a patient, a physiological osmolality of from 375 mOsmol/kg to 475 mOsmol/kg is obtained in said patient.

8. The stable, aqueous composition of any one of claims 1-7, wherein the composition has a minimal amount of one or more of degradation products each selected from the group consisting of cyclo proline-threonine (diketopiperazine), Thr-Pro-Pro-Thr, Pro-Pro-Thr, Pro-Pro-Thr-$NH_2$, Thr-Pro, Pro-Thr, Pro-Thr-$NH_2$, proline and/or threonine after 10 days at room temperature, after 20 days at room temperature or after 1 month at 0 °C or below.

9. The stable, aqueous composition of any one of claims 1-8, wherein the composition has less than 2% area obtained by HPLC of the GLYX -13 peak of diketopiperazine and/or Pro-Thr-$NH_2$ after 3 months at 40 °C, preferably less than 1% or less than 0.5% area obtained by HPLC of the GLYX-13 peak by HPLC of diketopiperazine and/or Pro-Thr-NH2 after 3 weeks at 40 °C.

10. A receptacle containing an amount of the stable, aqueous composition of any one of claims 1-9, extractable as at least one single dose.

11. The receptacle of claim 10, wherein the single dose has a volume of 1 mL to 4 mL, and preferably has a volume of 3 mL.

12. A pre-filled syringe or vial comprising a single dose of the stable, aqueous liquid composition of any one of claims 1-9.

13. A stable, aqueous composition according to any one of claims 1-9 comprising:
150 mg/mL of a compound represented by:

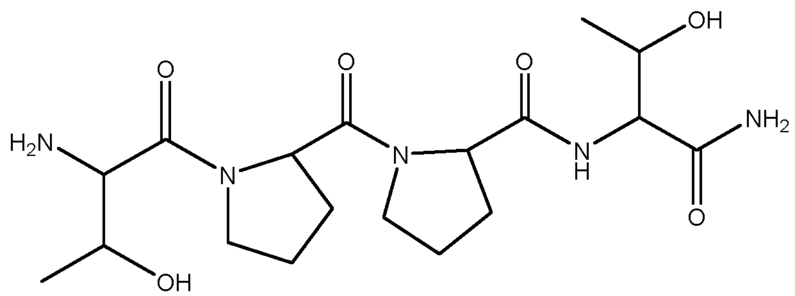

;

water for injection;
and hydrochloric acid, wherein the composition has a pH of 4.1 to 4.7 at 25°C.

14. A pharmaceutically acceptable dose suitable for injection comprising:
225 mg or 450 mg of a compound represented by:

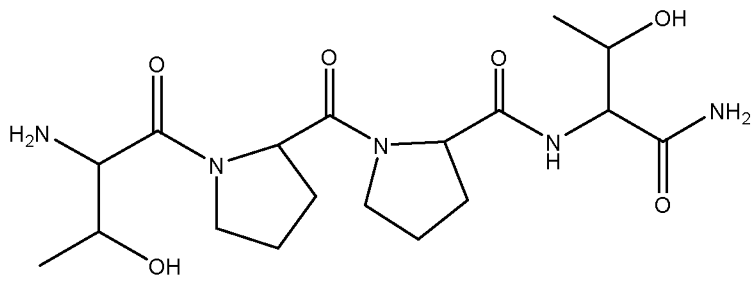

;

water; and
an acid providing chloride ions in the aqueous composition, wherein the dose has a pH of 4.5 and a volume of 3 mL.

**15.** The dose of claim 14, wherein the dose is disposed within a syringe or a vial.

**16.** The composition according to any one of claims 1-9 or 13, wherein the composition is prepared by a process comprising:

(i) providing a first combination comprising the pharmaceutically active compound and water; and
(ii) contacting the first combination with hydrochloric acid, or a source thereof, in an amount sufficient to achieve a pH of from 3.9 to 5.5.

**17.** The composition according to any one of claims 1-9 or 13 for the use of treating depression in a patient in need thereof.

**Patentansprüche**

**1.** Stabile, wässrige Zusammensetzung, die zur intravenösen Injektion geeignet ist, umfassend:
60 mg/mL bis 200 mg/mL einer pharmazeutisch aktiven Verbindung der Formel:

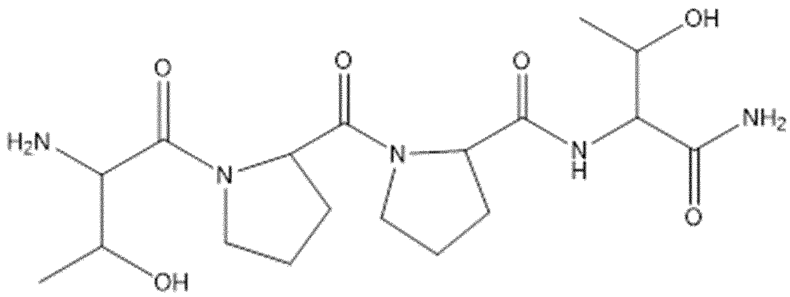

oder ein pharmazeutisch verträgliches Salz davon; Wasser zur Injektion; und
eine Säure;
wobei die stabile, wässrige Zusammensetzung bei 25 °C einen pH von 3,9 bis 5,5 aufweist; und
wobei die Säure Chloridionen in der wässrigen Zusammensetzung bereitstellt.

**2.** Stabile, wässrige Zusammensetzung gemäß Anspruch 1, umfassend 75 mg/mL, 150 mg/mL oder 125 mg/mL bis 175 mg/mL der pharmazeutisch aktiven Verbindung.

**3.** Stabile, wässrige Zusammensetzung gemäß einem der Ansprüche 1-2, umfassend 200 mg bis 500 mg der pharmazeutisch aktiven Verbindung, bevorzugt 225 mg, 375 mg oder 450 mg der pharmazeutisch aktiven Verbindung.

**4.** Stabile, wässrige Zusammensetzung gemäß einem der Ansprüche 1-3, wobei die stabile, wässrige Zusammensetzung bei 25 °C einen pH von 4,5 aufweist.

**5.** Stabile, wässrige Zusammensetzung gemäß einem der Ansprüche 1-4, umfassend mindestens eines von: $H^+$, einer protonierten Form der pharmazeutisch aktiven Verbindung und/oder eine Kombination davon.

**6.** Stabile, wässrige Zusammensetzung gemäß einem der Ansprüche 1-5, wobei nach Verabreichung einer Dosis der stabilen, wässrigen, flüssigen Zusammensetzung, die 150 mg/mL der pharmazeutisch aktiven Verbindung umfasst und ein Volumen von 3 mL aufweist, an einen Patienten, in besagtem Patienten eine physiologische Osmolalität von 800 mOsmol/kg bis 900 mOsmol/kg erzielt wird.

**7.** Stabile, wässrige Zusammensetzung gemäß einem der Ansprüche 1-5, wobei nach Verabreichung einer Dosis der stabilen, wässrigen, flüssigen Zusammensetzung, die 75 mg/mL der pharmazeutisch aktiven Verbindung umfasst und ein Volumen von 3 mL aufweist, an einen Patienten, in besagtem Patienten eine physiologische Osmolalität von 375 mOsmol/kg bis 475 mOsmol/kg erzielt wird.

**8.** Stabile, wässrige Zusammensetzung gemäß einem der Ansprüche 1-7, wobei die Zusammensetzung nach 10 Tagen bei Raumtemperatur, nach 20 Tagen bei Raumtemperatur oder nach 1 Monat bei 0°C oder darunter, eine minimale Menge eines oder mehrerer Abbauprodukte aufweist, die jeweils ausgewählt sind aus der Gruppe bestehend aus Cyclo-Prolin-Threonin (Diketopiperazin), Thr-Pro-Pro-Thr, Pro-Pro-Thr, Pro-Pro-Thr-$NH_2$, Thr-Pro, Pro-Thr, Pro-Thr-$NH_2$, Prolin und/oder Threonin.

**9.** Stabile, wässrige Zusammensetzung gemäß einem der Ansprüche 1-8, wobei die Zusammensetzung nach 3 Monaten bei 40 °C in der HPLC einen GLYX-13-Peak von Diketopiperazin und/oder Pro-Thr-NH2 mit einer Fläche von weniger als 2% aufweist, bevorzugt nach 3 Wochen bei 40 °C in der HPLC einen GLYC-13-Peak von Diketopiperazin und/oder Pro-Thr-NH2 mit einer Fläche von weniger als 1% oder weniger als 0,5% aufweist.

**10.** Behälter, enthaltend eine Menge der stabilen, wässrigen Zusammensetzung gemäß einem der Ansprüche 1-9, entnehmbar als mindestens eine Einzeldosis.

**11.** Behälter gemäß Anspruch 10, wobei die Einzeldosis ein Volumen von 1 mL bis 4 mL aufweist, und bevorzugt ein Volumen von 3 mL aufweist.

**12.** Vorgefüllte Spritze oder Fläschchen, umfassend eine Einzeldosis der stabilen, wässrigen, flüssigen Zusammensetzung gemäß einem der Ansprüche 1-9.

**13.** Stabile, wässrige Zusammensetzung gemäß einem der Ansprüche 1-9, umfassend:
150 mg/mL einer Verbindung dargestellt durch:

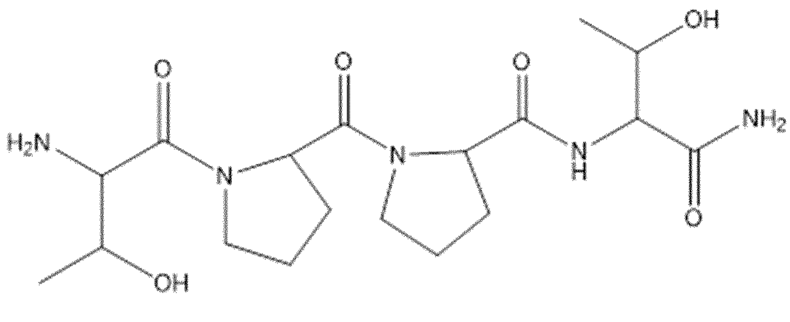

Wasser zur Injektion; und
Salzsäure,
wobei die Zusammensetzung bei 25 °C einen pH von 4,1 bis 4,7 aufweist.

**14.** Pharmazeutisch verträgliche Dosis, die zur Injektion geeignet ist, umfassend:
225 mg oder 450 mg einer Verbindung dargestellt durch:

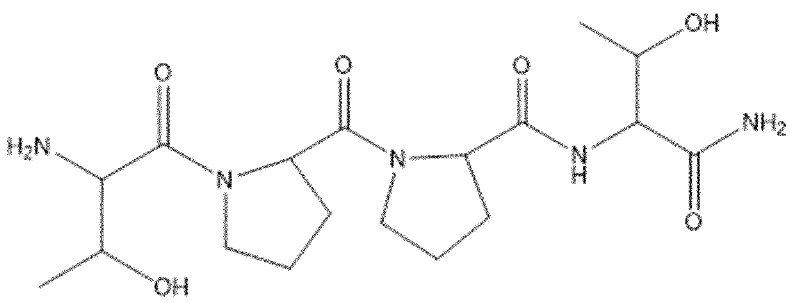

Wasser; und
eine Säure die Chloridionen in der wässrigen Zusammensetzung bereitstellt,
wobei die Dosis einen pH von 4,5 und ein Volumen von 3 mL aufweist.

15. Dosis gemäß Anspruch 14, wobei die Dosis in einer Spritze oder einem Fläschchen enthalten ist.

16. Zusammensetzung gemäß einem der Ansprüche 1-9 oder 13, wobei die Zusammensetzung durch einen Prozess hergestellt wird, umfassend:

(i) Bereitstellung einer ersten Kombination umfassend die pharmazeutisch aktive Verbindung und Wasser; und
(ii) Kontaktieren der ersten Kombination mit Salzsäure oder einer Quelle davon, in einer Menge, die ausreicht, um einen pH von 3,9 bis 5,5 zu erreichen.

17. Zusammensetzung gemäß einem der Ansprüche 1-9 oder 13 zur Verwendung zur Behandlung von Depressionen bei einem Patienten, der diese benötigt.

**Revendications**

1. Composition aqueuse stable adaptée à une injection intraveineuse, comprenant :
60 mg/mL à 200 mg/mL d'un composé pharmaceutiquement actif présentant la formule :

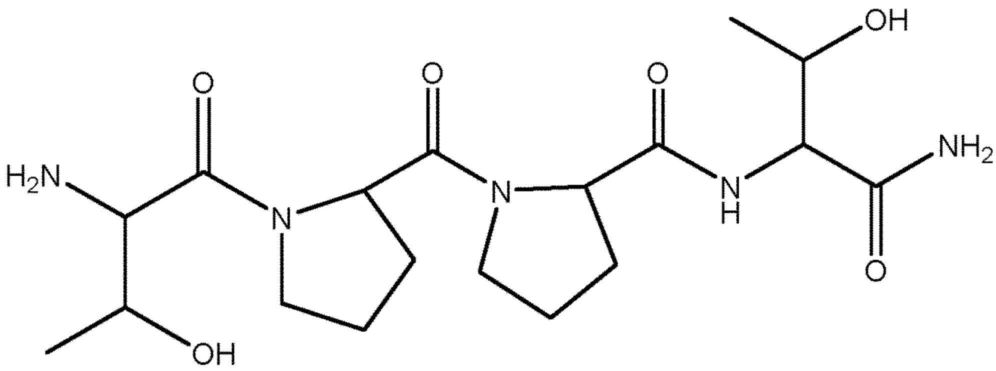

;

ou un sel pharmaceutiquement acceptable de celui-ci ;
de l'eau pour l'injection ; et
un acide ; dans laquelle la composition aqueuse stable présente un pH de 3,9 à 5,5 à 25 °C ; et
dans laquelle l'acide fournit des ions de chlorure dans la composition aqueuse.

2. Composition aqueuse stable selon la revendication 1, comprenant de 75 mg/mL à 150 mg/mL, ou de 125 mg/mL à 175 mg/mL du composé pharmaceutiquement actif.

3. Composition aqueuse stable selon l'une quelconque des revendications 1-2, comprenant 200 mg à 500 mg du composé pharmaceutiquement actif, de préférence, 225 mg, 375 mg ou 450 mg du composé pharmaceutiquement actif.

4. Composition aqueuse stable selon l'une quelconque des revendications 1-3, dans laquelle la composition aqueuse stable présente un pH de 4,5 à 25 °C.

5. Composition aqueuse stable selon l'une quelconque des revendications 1-4, comprenant au moins un de : $H^+$, une forme protonée du composé pharmaceutiquement actif et/ou une combinaison de ceux-ci.

6. Composition aqueuse stable selon l'une quelconque des revendications 1-5, dans laquelle lors de l'administration d'une dose de la composition liquide aqueuse stable qui comprend 150 mg/mL du composé pharmaceutiquement actif et présente un volume de 3 mL à un patient, une osmolalité physiologique de 800 mOsmol/kg à 900 mOsmol/kg est obtenue dans ledit patient.

7. Composition aqueuse stable selon l'une quelconque des revendications 1-5, dans laquelle lors de l'administration d'une dose de la composition liquide aqueuse stable qui comprend 75 mg/mL du composé pharmaceutiquement actif et présente un volume de 3 mL à un patient, une osmolalité physiologique de 375 mOsmol/kg à 475 mOsmol/kg est obtenue dans ledit patient.

8. Composition aqueuse stable selon l'une quelconque des revendications 1-7, dans laquelle la composition présente une quantité minimale d'un ou de plusieurs produits de dégradation, chacun étant choisi dans le groupe consistant en cyclo proline-thréonine (dicétopipérazine), Thr-Pro-Pro-Thr, Pro-Pro-Thr, Pro-Pro-Thr-$NH_2$, Thr-Pro, Pro-Thr, Pro-Thr-$NH_2$, proline et/ou thréonine au bout de 10 jours à température ambiante, après 20 jours à température ambiante ou après 1 mois à 0 °C ou en dessous.

9. Composition aqueuse stable selon l'une quelconque des revendications 1-8, dans laquelle la composition présente moins de 2% de surface obtenue par CLHP du pic GLYX-13 de dicétopipérazine et/ou de Pro-Thr-$NH_2$ au bout de 3 mois à 40°C, de préférence moins de 1 % ou moins de 0,5 % de surface obtenue par CLHP du pic GLYX-13 par CLHP de dicétopipérazine et/ou de Pro-Thr-NH2 au bout de 3 semaines à 40 °C.

10. Réceptacle contenant une quantité de la composition aqueuse stable selon l'une quelconque des revendications 1-9, extractible sous la forme d'au moins une dose unique.

11. Réceptacle selon la revendication 10, dans lequel la dose unique présente un volume de 1 mL à 4 mL, et présente de préférence un volume de 3 mL.

12. Seringue ou flacon pré-rempli(e) comprenant une dose unique de la composition liquide aqueuse stable selon l'une quelconque des revendications 1-9.

13. Composition aqueuse stable selon l'une quelconque des revendications 1-9 comprenant :
150 mg/mL d'un composé représenté par :

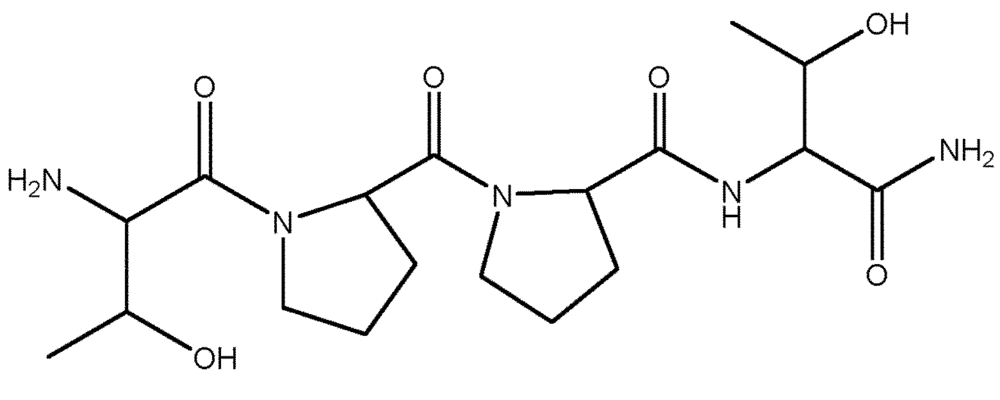
;

de l'eau pour l'injection ; et
de l'acide chlorhydrique, dans laquelle la composition présente un pH de 4,1 à 4,7 à 25 °C.

14. Dose pharmaceutiquement acceptable adaptée à une injection comprenant :
225 mg ou 450 mg d'un composé représenté par :

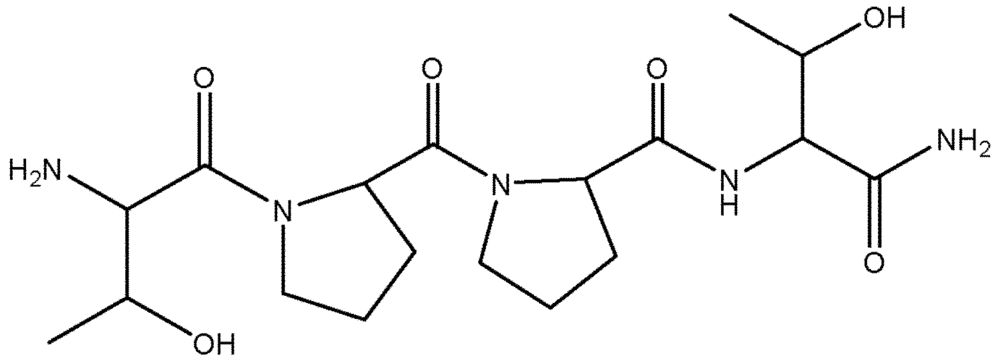

;

de l'eau ; et
un acide fournissant des ions de chlorure dans la composition aqueuse, dans laquelle la dose présente un pH de 4,5 et un volume de 3 mL.

**15.** Dose selon la revendication 14, dans laquelle la dose est disposée à l'intérieur d'une seringue ou d'un flacon.

**16.** Composition selon l'une quelconque des revendications 1-9 ou 13, dans laquelle la composition est préparée par un procédé comprenant :

(i) la fourniture d'une première combinaison comprenant le composé pharmaceutiquement actif et de l'eau ; et
(ii) la mise en contact de la première combinaison avec de l'acide chlorhydrique, ou une source de celui-ci, dans une quantité suffisante pour obtenir un pH de 3,9 à 5,5.

**17.** Composition selon l'une quelconque des revendications 1-9 ou 13 pour une utilisation dans le traitement d'une dépression chez un patient en présentant besoin.

**Figure 1. shows the formation of Impurity 1 (% area proline-threonine diketopiperazine, RRT 0.43) over time versus pH.**

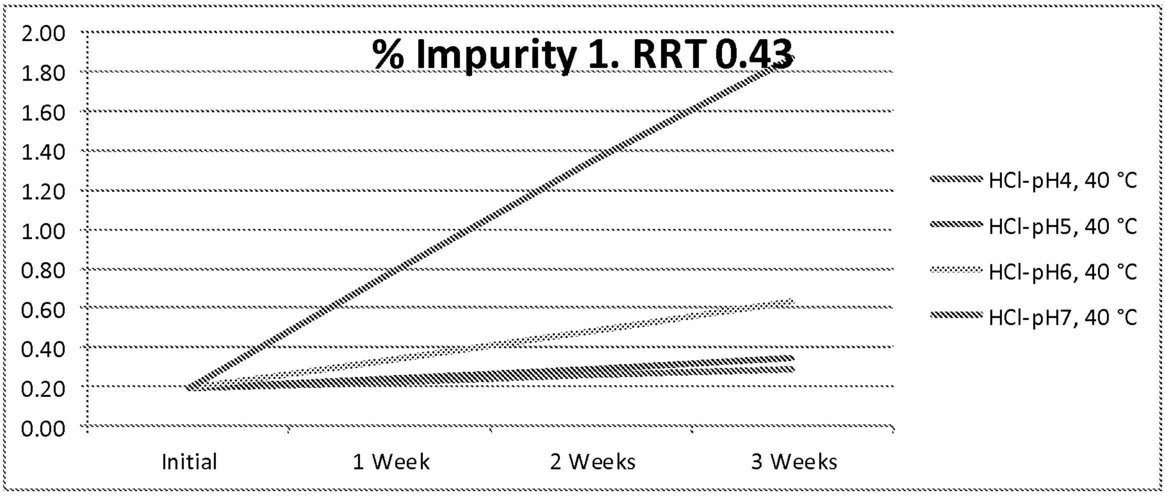


**Figure 2. Formation of Impurity 2 (% area proline-threonine-amide, RRT 0.57) over time versus pH.**

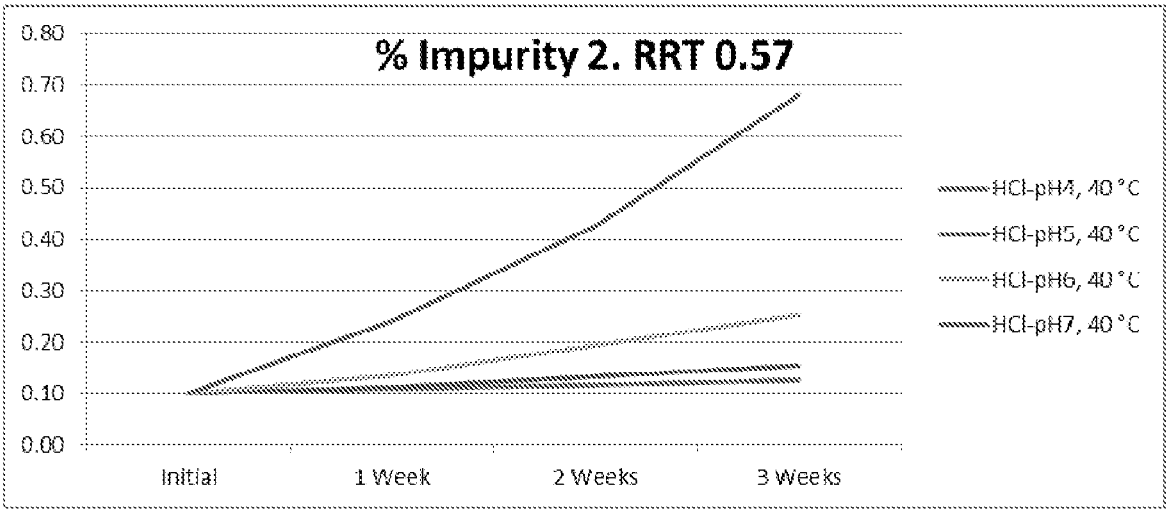

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5763393 A
- US 4086196 A


### Non-patent literature cited in the description


- **COOPER ; BLOOM ; ROTH.** The Biochemical Basis of Neuropharmacology. Oxford University Press, 1986
- **BLISS ; COLLINGRIDGE.** *Nature,* 1993, vol. 361, 31-39